(19) Europäisches Patentamt
European Patent Office
Office européen des brevets

(11) Veröffentlichungsnummer: **0 461 487 A2**

# (12) EUROPÄISCHE PATENTANMELDUNG

(21) Anmeldenummer: **91108913.4**

(22) Anmeldetag: **31.05.91**

(51) Int. Cl.5: **C07D 213/30**, C07D 213/48, A01N 43/40, C07D 213/50, C07D 213/26, C07D 213/53

(30) Priorität: **07.06.90 DE 4018260**

(43) Veröffentlichungstag der Anmeldung: **18.12.91 Patentblatt 91/51**

(84) Benannte Vertragsstaaten: **AT BE CH DE DK ES FR GB GR IT LI NL SE**

(71) Anmelder: **BASF Aktiengesellschaft**
**Carl-Bosch-Strasse 38**
**W-6700 Ludwigshafen(DE)**

(72) Erfinder: **Wagner, Oliver, Dr.**
**Karlsbader Strasse 7**
**W-6700 Ludwigshafen(DE)**
Erfinder: **Zipperer, Bernhard, Dr.**
**Am Herrgottsacker 6**
**W-6716 Dirmstein(DE)**
Erfinder: **Goetz, Norbert, Dr.**
**Schoefferstrasse 25**
**W-6520 Worms 1(DE)**
Erfinder: **Keil, Michael, Dr.**
**Fontanestrasse 4**
**W-6713 Freinsheim(DE)**
Erfinder: **Ammermann, Eberhard, Dr.**
**Sachsenstrasse 3**
**W-6700 Ludwigshafen(DE)**
Erfinder: **Lorenz, Gisela, Dr.**
**Erlenweg 13**
**W-6730 Neustadt(DE)**

## (54) Derivate des beta-Picolins und diese enthaltende Pflanzenschutzmittel.

(57) β-Picolinderivate der Formel

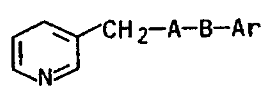

in der

A die Gruppe $CR^1R^2$ bedeutet, wobei $R^1$, $R^2$ unabhängig voneinander für Wasserstoff, Alkyl, Alkenyl, Alkinyl stehen oder $R^1$ und $R^2$ zusammen eine Methylenkette bedeuten,

B eine der Gruppen $CH_2$, $CHOR^3$, $CHalR^4$, C=O, C=N-O-$R^5$ bedeutet, wobei $R^3$ für Wasserstoff, Alkyl, Halogenalkyl, Cycloalkyl, Alkenyl, Acyl, Phenyl, Benzyl, Benzoyl, steht, wobei der Phenylring ggf. substituiert sein kann, $R^4$ Wasserstoff, Fluor, Chlor, Brom oder Jod, $R^5$ Wasserstoff, Alkyl, Halogenalkyl, Cycloalkyl, Alkenyl, Phenyl, Benzyl bedeutet, wobei der Phenylring ggf. substituiert ist,

Ar einen Arylrest bedeutet, der ggf. substituiert ist,

und ihre N-Oxide und pflanzenverträglichen Säureadditionssalze und diese Verbindungen enthaltende Fungizide.

EP 0 461 487 A2

Die vorliegende Erfindung betrifft β-Picolinderivate und diese enthaltende Fungizide.

Die Verbindung 1-Phenyl-3-(3-pyridinyl)propan-1-on ist aus J. Org. Chem. 43 (1978), 3396 und aus Arch. Pharm. 307 (1974), 550 bekannt, jedoch wird nichts über eine fungizide Wirkung berichtet.

Es wurde gefunden, daß β-Picolinderivate der Formel

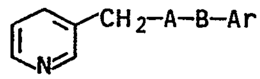

(I)

in der

A die Gruppe $CR^1R^2$ bedeutet, wobei
$R^1$, $R^2$ unabhängig voneinander für Wasserstoff, $C_1$-$C_6$-Alkyl, $C_2$-$C_6$-Alkenyl, $C_2$-$C_6$-Alkinyl stehen oder $R^1$ und $R^2$ zusammen eine Methylenkette mit 2 bis 6 Methylengruppen bedeuten,

B eine der Gruppen $CH_2$, $CHOR^3$, $CHR^4$, C=O, $C=N-O-R^5$ bedeutet, wobei
$R^3$ für Wasserstoff, $C_1$-$C_6$-Alkyl, $C_1$-$C_6$-Halogenalkyl, $C_3$-$C_8$-Cycloalkyl, $C_3$-$C_6$-Alkenyl, $C_2$-$C_6$-Acyl, Phenyl, Benzyl, Benzoyl steht, wobei der Phenylring ggf. durch ein bis drei Substituenten aus der Gruppe $C_1$-$C_4$-Alkyl, $C_1$-$C_4$-Alkoxy, $C_1$-$C_4$-Halogenalkyl, $C_1$-$C_4$-Halogenalkoxy, Halogen, Cyan oder Nitro substituiert sein kann,
$R^4$ Wasserstoff, Fluor, Chlor, Brom oder Jod,
$R^5$ Wasserstoff, $C_1$-$C_6$-Alkyl, $C_1$-$C_6$-Halogenalkyl, $C_3$-$C_7$-Cycloalkyl, $C_3$-$C_6$-Alkenyl, Aralkyl mit 1 bis 4 Kohlenstoffatomen im Alkylrest bedeutet, wobei der Arylrest gegebenenfalls durch ein bis drei Substituenten aus der Gruppe $C_1$-$C_4$-Alkyl, $C_1$-$C_4$-Alkoxy, $C_1$-$C_4$-Halogenalkyl, Halogen, Cyan oder Nitro substituiert sein kann,

Ar einen ein- bis zweikernigen Arylrest bedeutet, der ggf. ein bis dreifach substituiert ist durch Halogen, $C_1$-$C_6$-Alkyl, $C_1$-$C_4$-Alkoxy, $C_1$-$C_4$-Halogenalkyl, $C_1$-$C_4$-Halogenalkoxy, Phenyl, Phenoxy, Halogenphenyl, Halogenphenoxy oder Benzyloxy,

und ihre N-Oxide und pflanzenverträglichen Säureadditionssalze, außer der Verbindung, in der A $CH_2$, B C=O und Ar Phenyl bedeuten, eine gute fungizide Wirkung haben, die besser als die Wirkung bekannter Wirkstoffe ist.

$R^1$, $R^2$ bedeuten beispielsweise jeweils unabhängig voneinander Wasserstoff, Methyl, Ethyl, n-Propyl, Isopropyl, n-Butyl, Isobutyl, tert.-Butyl, n-Pentyl, neo-Pentyl, iso-Pentyl, n-Hexyl, Allyl, 2-Methylallyl, 3-Methylallyl, 3,3-Dimethylallyl, Propargyl. Bevorzugt werden die Reste $CR^1R^2$, in denen $R^1$ verschieden von Wasserstoff ist, insbesondere die Reste, in denen $R^1$ und $R^2$ verschieden von Wasserstoff sind.

$R^1$ und $R^2$ zusammen können ferner zusammen mit dem C-Atom, dessen Substituenten sie sind, einen Cycloalkylring mit 3 bis 7 C-Atomen bilden, der 2 bis 6 Methylengruppen enthält.

$R^3$ bedeutet beispielsweise Wasserstoff, Methyl, Ethyl, n-Propyl, Isopropyl, n-Butyl, Isobutyl, tert.-Butyl, n-Pentyl, neo-Pentyl, iso-Pentyl, n-Hexyl, Cyclopropyl, Cyclopentyl, Cyclohexyl, Cycloheptyl, Cyclooctyl, Allyl, 2-Methylallyl, 3-Methylallyl, 3,3-Dimethylallyl, Propargyl, Trifluormethyl, Chlormethyl, Dichlormethyl, Trichlormethyl, Brommethyl, 2-Bromethyl, 2-Chlorethyl, 3-Brompropyl, 4-Brombutyl, Phenyl, Mono-, Di-, Trimethylphenyl, 4-tert. Butylphenyl, Mono-, Di-, Trimethoxyphenyl, Trifluormethylphenyl, Fluorphenyl, Mono-, Di-, Trichlorphenyl, Mono-, Dinitrophenyl, Cyanphenyl, Mono-, Di-, Tribenzyl, 4-tert. Butylphenyl, Mono-, Di-, Trimethoxybenzyl, Trifluormethylbenzyl, Fluorbenzyl, Mono-, Di-, Trichlorbenzyl, Mono-, Dinitrobenzyl, Cyanobenzyl, Mono-, Di-, Trimethylbenzoyl, 4-tert.-Butylbenzoyl, Mono-, Di-, Trimethoxybenzoyl, Trifluormethylbenzoyl, Fluorbenzoyl, Mono-, Di-, Trichlorbenzoyl, Mono-, Dinitrobenzoyl, Cyanobenzoyl, Acetyl, Propionyl, Butyryl, Pentyryl, Hexyryl.

$R^5$ bedeutet beispielsweise Wasserstoff, Methyl, Ethyl, n-Propyl, Iso-propyl, n-Butyl, Isobutyl, tert.-Butyl, n-Pentyl, neo-Pentyl, iso-Pentyl, n-Hexyl, Cyclopropyl, Cyclobutyl, Cyclopentyl, Cyclohexyl, Cycloheptyl, Cyclooctyl, Allyl, 2-Methylallyl, 3-Methylallyl, 3,3-Dimethylallyl, Propargyl, Trifluormethyl, Chlormethyl, Dichlormethyl, Trichlormethyl, Brommethyl, 2-Bromethyl, 2-Chlorethyl, 3-Brompropyl, 4-Brombutyl, Phenyl, Mono-, Di-, Trimethylphenyl, 4-tert. Butylphenyl, Mono-, Di-, Trimethoxyphenyl, Trifluormethylphenyl, Fluorphenyl, Mono-, Di-, Trichlorphenyl, Mono-, Dinitrophenyl, Cyanphenyl, Mono-, Di-, Trimethylbenzyl, 4-tert.-Butylbenzyl, Mono-, Di-, Trimethoxybenzyl, Trifluormethylbenzyl, Fluorbenzyl, Mono-, Di-, Trichlorbenzyl, Mono-, Dinitrobenzyl, Cyanbenzyl, Ar bedeutet beispielsweise Phenyl, 2-Methylphenyl, 3-Methylphenyl, 4-Methylphenyl, 2,4-Dimethylphenyl, 2,6-Dimethylphenyl, 2,4,6-Trimethylphenyl, 4-Isopropylphenyl, 4-tert.-Butylphenyl, 1-Naphthyl, 2-Napthyl, 4-Biphenyl, 2-Fluorphenyl, 3-Fluorphenyl, 4-Fluorphenyl, 2,4-Difluorphenyl, 2-Chlorphenyl, 3-Chlorphenyl, 4-Chlorphenyl, 2,4-Dichlorphenyl, 3,4-Dichlorphenyl, 2,4,6-Trichlorphenyl, 2-Chlor-4-Fluorphenyl, 2-Trifluormethylphenyl, 3-Trifluormethylphenyl, 4-Trifluormethylphenyl, 2-, 3-, oder 4-Methoxyphenyl, 3,4-Dimethoxyphenyl, 4-Trifluormethoxyphenyl, 4-Tetrafluorethoxyphenyl, 2-Chlor-4-(4'-chlorphenoxy)phenyl, 4-Phenoxyphenyl, 4-(4'-Chlorphenoxy)phenyl, 4-Benzyloxyphenyl.

Die Verbindungen der Formel I können gegebenenfalls zwei oder mehrere Asymmetrie-Zentren aufweisen und daher in zwei oder mehreren diastereomeren Formen vorliegen, welche durch bekannte Verfahren, beispielsweise durch Chromatographie oder Kristallisation getrennt werden können. Die vorliegende Erfindung umfaßt sowohl die reinen Diastereomeren als auch deren Mischungen und ihre Anwendung als Fungizide.

Die Herstellung der $\beta$-Picolinderivate kann in der Weise erfolgen, daß man einen Aldehyd der Formel II

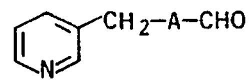

(II)

in der A die oben genannten Bedeutungen hat, mit einer Organometallverbindung der Formel III

Ar-M (III)

in der M für Lithium oder einen der Reste MgCl, MgBr oder MgJ steht und Ar die oben genannten Bedeutungen hat, umsetzt. Dabei ist es von Vorteil, die Organometallverbindung der Formel III in einem inerten Lösungsmittel, vorzugsweise Ether wie Diethylether oder Tetrahydrofuran bei Temperaturen von 0°C bis 80°C bevorzugt von 0°C bis 20°C vorzulegen und den Aldehyd der Formel II gegebenenfalls in einem Verdünnungsmittel gelöst, zuzutropfen.

Die metallorganischen Verbindungen der Formel III sind allgemein bekannt. Die Aldehyde der Formel II, in der A für $CH_2$ oder $CH(CH_3)$ steht, sind aus J. Org. Chem. 43 (1978), 3396 und 2947 bekannt. Die übrigen Aldehyde der Formel II, in der A die oben genannten Bedeutungen hat, sind neu. Sie können beispielsweise dadurch hergestellt werden, daß man einen Aldehyd der Formel IV

H-A-CHO (IV)

in der A die oben genannten Bedeutungen mit Ausnahme von $CH_2$ und $CH(CH_3)$ hat, mit in situ erzeugtem 3-Chlormethylpyridin in Gegenwart einer Base alkyliert. 3-Chlormethylpyridin ist unbeständig und neigt schon bei Raumtemperatur zur Verharzung (vgl. z.B. CA 47, 8068e). Ein in situ aus dem Hydrochlorid freigesetztes 3-Chlormethylpyridin ist ein zur Alkylierung von Aldehyden geeignetes Reagenz.

Die direkte alpha-Alkylierung von Aldehyden verläuft in der Regel nur mit schlechten Ausbeuten, gleiches gilt auch für die von den Aldehyden abgeleiteten Enamine (vgl. z.B. G. Opitz et al. Liebigs Ann. Chem. 649 (1961) 36. Eine präparativ einfache Methode ist die Phasentransfer-katalysierte alpha-Alkylierung von Aldehyden. Dieses Verfahren kann als fest/flüssig-Variante in einem Zweiphasensystem, bestehend aus festem Natriumhydroxid und einem lipophilen organischen Lösungsmittel (vgl. H.K. Dietl, K.C. Brannock, Tetrahedron Lett. 1273 (1973); E. Buschmann, B. Zeeh, Liebigs Ann. Chem. 1585 (1979) durchgeführt werden. Besonders vorteilhaft ist hier eine flüssig-flüssig-Variante, bei der die Natronlauge und ein organisches, nicht mit Wasser mischbares Lösungsmittel, sowie ein geeigneter Phasentransfer-Katalysator vorgelegt und der Aldehyd IV, ggf. gelöst in einem Verdünnungsmittel und das 3-Chlormethylpyridin-Hydrochlorid, ggf. als wässrige Lösung zudosiert werden. Als organische Phase eignen sich beispielsweise Kohlenwasserstoffe wie Petrolether, Cyclohexan, Benzol, Toluol, Xylol oder chlorierte Kohlenwasserstoffe wie Dichlormethan, 1,2-Dichlorethan. Als Phasentransfer-Katalysator können beispielsweise Kronenether oder quartäre Ammoniumsalze, vorzugsweise Tetra-n-butylammoniumsalze, Benzyltriethylammoniumsalze oder Methyltrioctylammoniumsalze eingesetzt werden. Die Reaktion wird bevorzugt bei 0°C bis 100°C, insbesondere bei 20°C bis 80°C durchgeführt. Ein weiteres Verfahren zur Herstellung von Aldehyden der Formel II ist dadurch gekennzeichnet, daß man einen $\alpha,\beta$-ungesättigten Aldehyd der Formel

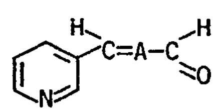

(V)

in der A für C-($C_1$-$C_6$-alkyl) steht, mit Wasserstoff in Gegenwart eines geeigneten Katalysators hydriert. Die

Aldehyde der Formel V sind neu. Ihre Herstellung kann in der Weise erfolgen, daß man den Aldehyd

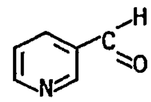

mit einem Aldehyd

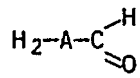

umsetzt (analog EP 298 380).

Die Verfahren zur Durchführung der Oxidation der Alkohole der allgemeinen Formel I (B = CHOH) sind literaturbekannt (siehe z.B. Houben-Weyl, Methoden der organischen Chemie, Band VII/2a, Ketone Teil 1, S. 699ff). Als bevorzugtes Verfahren kommt die Oxidation mit Dimethylsulfoxid in Gegenwart geeigneter Hilfsreagenzien z.B. Oxalylchlorid/Triethylamin zur Anwendung. Als Verdünnungsmittel kommen inerte organische Lösungsmittel z.B. Kohlenwasserstoffe wie Petrolether, Cyclohexan, Benzol oder chlorierte Kohlenwasserstoffe wie Dichlormethan, 1,2-Dichlorethan, Chloroform oder Ether wie z.B. Diethylether, Tetrahydrofuran, Dioxan infrage. Die Reaktion wird in einem Temperaturbereich zwischen -80°C und 50°C bevorzugt -70°C bis -10°C durchgeführt.

Die Chloride der allgemeinen Formel I mit B = H-C-Cl sind aus den entsprechenden Hydroxydverbindungen durch literaturbekannte Verfahren zugänglich (vgl. z.B. Houben-Weyl, Methoden der organischen Chemie, Band V/3, Halogenverbindungen).

Als bevorzugtes Verfahren kommt dabei die Umsetzung von Thionylchlorid mit den Alkoholen zur Anwendung. Als Verdünnungsmittel kommen inerte Lösungsmittel, beispielsweise Kohlenwasserstoffe wie Petrolether, Cyclohexan, Benzol, Toluol, Xylol in Frage. Die Reaktion wird bei Temperaturen von 40°C bis 150°C vorzugsweise bei 80°C mit oder ohne Katalysator durchgeführt. Als Katalysatoren eignen sich beispielsweise DMF oder tertiäre Amine wie Triethylamin, N,N-Dimethylanilin oder Piperidin.

Oximether der allgemeinen Formel I mit B = C = $R^5$, wobei $R^5$ die im Anspruch genannten Bedeutungen hat, sind nach an sich bekannten Verfahren zugänglich (siehe Houben-Weyl, Methoden der organischen Chemie, X,4/55).

Als bevorzugtes Verfahren kommt die Umsetzung der Ketone der allgemeinen Formel I mit B = C = O und einem Hydroxylamin in einem geeigneten Verdünnungsmittel und geeigneter Hilfsreagenzien zur Anwendung. Als Verdünnungsmittel kommen inerte organische Lösungsmittel z.B. Kohlenwasserstoffe wie Petrolether, Cyclohexan, Benzol oder chlorierte Kohlenwasserstoffe wie Dichlormethan, 1,2-Dichlorethan, Chloroform oder Ether wie z.B. Diethylether, Tetrahydrofuran, Dioxan, Nitrile, wie Acetonitril, Propionitril und Alkohole wie Methanol, Ethanol und n-Butanol in Frage.

Als Reaktionshilfsmittel kommen alle gängigen anorganischen und organischen Basen in Frage, wie beispielsweise Alkalihydroxide: Natrium-Kaliumhydroxid, Alkalialkoholate: Natriummethanolat, -ethanolat, Kalium-tert.-butanolat, Alkalicarbonate: Natrium und Kaliumcarbonat, Natrium -Kaliumhydrogencarbonat; tertiäre Amine: Triethylamin, N,N-Dimethylanilin, Piperidin.

Die Reaktion wird bei Temperaturen von 20 bis 150°C, vorzugsweise zwischen 50 bis 130°C durchgeführt.

Die N-Oxide können in der Weise hergestellt werden, daß man die β-Picolinderivate der Formel I oxidiert.

Pflanzenverträgliche Säureadditionssalze sind Salze der β-Picolinderivate der Formel I mit anorganischen oder organischen Säuren, z.B. Schwefelsäure, Phosphorsäure, Essigsäure, Propionsäure, Oxalsäure, Phenylsulfonsäure, Dodecylbenzolsulfonsäure.

Die folgenden Vorschriften und Beispiele erläutern die Herstellung der Vorprodukte und der neuen Verbindungen

Vorschrift 1

2-(Pyridinyl-3-methyl)hexanol

In einem 0,3 l Rührautoklaven wird die Mischung von 21 g (0,11 mol) 2-Butyl-3-pyridinyl-propenal, 120 ml Methanol, 10 g N-Methylmorpholin und 6 g Hydrierkatalysator (10 % Pd, 5 % $Pr_2O_3$ auf $Al_2O_3$) bei 75°C und 75 bar Wasserstoffdruck bis zur Druckkonstanz hydriert. Die Lösung wird über Kieselgel abgesaugt, das Filtrat im Vakuum eingeengt und der Rückstand destillativ gereinigt.
Ausbeute: 24,1 g (56,8 %)

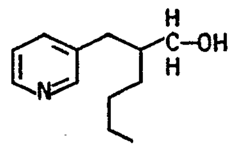

Vorschrift 2

2-(Pyridinyl-3-methyl)hexanal

Zu 17 g (0,132 mol) Oxalylchlorid in 280 ml $CH_2Cl_2$ tropft man bei -60°C 23 g (0,132 mol) Dimethylsulfoxid in 120 ml $CH_2Cl_2$ innerhalb einer Stunde zu. Anschließend gibt man 23 g (0,12 mol) 2-(Pyridinyl-3-methyl)hexanol in 240 ml $CH_2Cl_2$ langsam zu und nach 30 min 62 g Triethylamin.

Man läßt langsam auf Raumtemperatur (RT, 20°C) kommen, gibt 350 ml $H_2O$ zu. Die wässrige Phase wird 3 x mit je 300 ml $CH_2Cl_2$ extrahiert und die vereinigten Methylenchloridphasen mit $NaHCO_3$-Lösung gewaschen. Nach dem Trocknen über $Na_2SO_4$ und Abdampfen des Lösungsmittels wird der Rückstand der Destillation unterworfen. Man erhält 8,2 g (36 % der Titelverbindung) (118°C-120°C, 0,9 mbar).

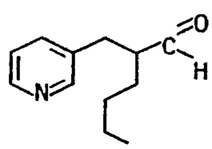

Vorschrift 3

2-Ethyl-2-(pyridinyl-3-methyl)butanal

Zu einem Gemisch von 450 ml Toluol, 600 ml 30 %ige (Gew.%) NaOH (4,5 mol) und 7,5 g Tetrabutylammoniumjodid tropft man bei 80°C die Lösung von 98,4 g (0,6 mol) $\beta$-Picolinchloridhydrochlorid, 66 g (0,66 mol) 2-Ethylbutanal in 600 ml Toluol innerhalb von 3 Stunden.

Man rührt anschließend weitere 3 h bei dieser Temperatur und gibt nach dem Abkühlen 1000 ml Toluol zu. Die organische Phase wird abgetrennt, 3 mal mit Wasser gewaschen, getrocknet und eingeengt.

Die Destillation (128°C-132°C/3 mbar) liefert 56 g der Titelverbindung (49 %).

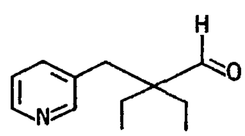

Beispiel 1

2-Ethyl-1-(4-Fluorphenyl)-2-(pyridinyl-3-methyl)butan-1-ol

Zu 2,8 g Mg-Späne (0,166 mol) tropft man 20,4 g (0,116 mol) 4-Fluor-brombenzol in 250 ml Tetrahydrofuran und rührt eine 1/2 h bei RT. Anschließend werden 11 g (0,058 mol) 2-Ethyl-2-(pyridinyl-3-methyl)butanal in 100 ml THF langsam zugetropft. Nach 2 h wird auf Eiswasser gegossen und mit gesättigter $NH_4Cl$-Lösung auf pH = 8 gestellt. Man extrahiert mit Ether, wäscht die organische Phase mit Wasser, trocknet über $Na_2SO_4$ und dampft anschließend das Lösungsmittel ab. Man erhält 8 g (48 %) der Titelverbindung als zähflüssiges Öl (Verbindung Nr. 70 in der Tabelle).

Beispiel 2

2-Ethyl-1-(4-Fluorphenyl)-2-(pyridinyl-3-methyl)butan-1-on

Zu 18,6 g Oxalylchlorid (0,144 mol) in 200 ml $CH_2Cl_2$ tropft man bei -60°C 22,5 g Dimethylsulfoxid (0,288 mol) in 50 ml Methylenchlorid. Man läßt bei -60°C 5 min nachreagieren.

Anschließend gibt man 33 g (0,115 mol) 2-Ethyl-1-(4-Fluorphenyl)-2-(pyridinyl-3-methyl)butanol in 100 ml $CH_2Cl_2$ langsam dazu und fügt nach 15 min 58,2 g (0,576 mol) Triethylamin zu. Man läßt langsam auf RT kommen, gibt 350 ml Wasser zu, extrahiert die wässrige Phase 3 x mit je 200 ml $CH_2Cl_2$ und trocknet die vereinigten organischen Phasen über $Na_2SO_4$. Nach dem Abdampfen des Lösungsmittels fällt die Titelverbindung in 49 % Ausbeute an (Verbindung Nr. 331).

Beispiel 3

1-Chlor-2-ethyl-1-phenyl-2-(pyridinyl-3-methyl)butan

Zu 5 g (0,018 mol) 2-Ethyl-1-(phenyl)-2-pyridinyl-3-methyl)butanol in 100 ml Toluol und einer katalytischen Menge Dimethylformamid (0,1 g), tropft man bei 80°C 13,3 g (0,11 mol) $SOCl_2$ und beläßt 5 h bei dieser Temperatur. Nach dem Abkühlen gießt man auf Eiswasser und stellt mit 30 % NaOH-Lösung alkalisch. Die wässrige Phase wird 3 mal mit je 50 ml tert.-Butylmethylether extrahiert und die vereinigten organischen Phasen über $MgSO_4$ getrocknet. Nach dem Abdampfen des Lösungsmittels wird der Rückstand an Kieselgel chromatographiert (Toluol/Essigester = 1:1). Man erhält 2,07 g der Titelverbindung als zähes gelbes Öl (Ausbeute 40 %) (Verbindung Nr. 581).

In entsprechender Weise können die in der folgenden Tabelle aufgeführten Verbindungen hergestellt werden.

Tabelle

| Nr. | A | B | Ar | phys. Daten | IR, $cm^{-1}$ |
|---|---|---|---|---|---|
| 1 | $C(CH_3)_2$ | CHOH | Phenyl | öl | 2963,1449,1424,1361,1059,1045 |
| 2 | $C(CH_3)_2$ | CHOH | 2-Methylphenyl | | 107-110°C |
| 3 | $C(CH_3)_2$ | CHOH | 4-Methylphenyl | öl | 3146,1474,1426,1069,1045,824 |
| 4 | $C(CH_3)_2$ | CHOH | 2,4-Dimethylphenyl | | |
| 5 | $C(CH_3)_2$ | CHOH | 2,6-Dimethylphenyl | | |
| 6 | $C(CH_3)_2$ | CHOH | 2,4,6-Trimethylphenyl | | 155-157°C |
| 7 | $C(CH_3)_2$ | CHOH | 4-tert. Butylphenyl | | 144-146°C |
| 8 | $C(CH_3)_2$ | CHOH | 1-Naphthyl | | |
| 9 | $C(CH_3)_2$ | CHOH | 2-Naphthyl | | |
| 10 | $C(CH_3)_2$ | CHOH | 4-Biphenyl | | |
| 11 | $C(CH_3)_2$ | CHOH | 2-Fluorphenyl | | |
| 12 | $C(CH_3)_2$ | CHOH | 4-Fluorphenyl | öl | 3200,2966,2871,1602,1508,1424 |
| 13 | $C(CH_3)_2$ | CHOH | 2,4-Difluorphenyl | | |
| 14 | $C(CH_3)_2$ | CHOH | 2-Chlorphenyl | | |
| 15 | $C(CH_3)_2$ | CHOH | 3-Chlorphenyl | | |
| 16 | $C(CH_3)_2$ | CHOH | 4-Chlorphenyl | öl | 3200,2966,2934,2870,1486,1424 |
| 17 | $C(CH_3)_2$ | CHOH | 2,4-Dichlorphenyl | öl | 3300,2967,1587,1467,717 |
| 18 | $C(CH_3)_2$ | CHOH | 3,4-Dichlorphenyl | | |
| 19 | $C(CH_3)_2$ | CHOH | 2-Chlor-4-fluorphenyl | | |
| 20 | $C(CH_3)_2$ | CHOH | 2-Trifluormethylphenyl | | |
| 21 | $C(CH_3)_2$ | CHOH | 4-Trifluormethylphenyl | | |
| 22 | $C(CH_3)_2$ | CHOH | 4-Methoxyphenyl | öl | 3250,2961,1610,1511,1247 |
| 23 | $C(CH_3)_2$ | CHOH | 3,4-Dimethoxyphenyl | | |
| 24 | $C(CH_3)_2$ | CHOH | 4-Tetrafluorethoxyphenyl | | |

Tabelle (Fortsetzung)

| Nr. | A | B | Ar | phys. Daten | IR, $cm^{-1}$ |
|---|---|---|---|---|---|
| 25 | $C(CH_3)_2$ | CHOH | 4-Trifluormethoxyphenyl | | |
| 26 | $C(CH_3)_2$ | CHOH | 2-Chlor-4-(4'-Chlorphenoxy)phenyl | | |
| 27 | $C(CH_3)_2$ | CHOH | 4-Phenoxyphenyl | | |
| 28 | $C(CH_3)_2$ | CHOH | 4-(4'-Chlorphenoxy)phenyl | | |
| 29 | $C(CH_3)_2$ | CHOH | 4-Benzyloxyphenyl | | |
| 30 | $C(CH_3)(CH_2CH_3)$ | CHOH | Phenyl | öl | 3200, 2964, 2936, 2877, 1463, 1452 |
| 31 | $C(CH_3)(CH_2CH_3)$ | CHOH | 2-Methylphenyl | | |
| 32 | $C(CH_3)(CH_2CH_3)$ | CHOH | 4-Methylphenyl | öl | 3200, 2964, 2936, 2877, 1478, 1463, 1424 |
| 33 | $C(CH_3)(CH_2CH_3)$ | CHOH | 2,4-Dimethylphenyl | | |
| 34 | $C(CH_3)(CH_2CH_3)$ | CHOH | 2,6-Dimethylphenyl | | |
| 35 | $C(CH_3)(CH_2CH_3)$ | CHOH | 2,4,6-Trimethylphenyl | | |
| 36 | $C(CH_3)(CH_2CH_3)$ | CHOH | 4-tert. Butylphenyl | | |
| 37 | $C(CH_3)(CH_2CH_3)$ | CHOH | 1-Naphthyl | | |
| 38 | $C(CH_3)(CH_2CH_3)$ | CHOH | 2-Naphthyl | | |
| 39 | $C(CH_3)(CH_2CH_3)$ | CHOH | 4-Biphenyl | | |
| 40 | $C(CH_3)(CH_2CH_3)$ | CHOH | 2-Fluorphenyl | | |
| 41 | $C(CH_3)(CH_2CH_3)$ | CHOH | 4-Fluorphenyl | öl | 3200, 2965, 2937, 1602, 1508, 1424 |
| 42 | $C(CH_3)(CH_2CH_3)$ | CHOH | 2,4-Difluorphenyl | | |
| 43 | $C(CH_3)(CH_2CH_3)$ | CHOH | 2-Chlorphenyl | | |
| 44 | $C(CH_3)(CH_2CH_3)$ | CHOH | 3-Chlorphenyl | | |
| 45 | $C(CH_3)(CH_2CH_3)$ | CHOH | 4-Chlorphenyl | öl | 3190, 2965, 2936, 2877, 1486, 1464 |
| 46 | $C(CH_3)(CH_2CH_3)$ | CHOH | 2,4-Dichlorphenyl | | |
| 47 | $C(CH_3)(CH_2CH_3)$ | CHOH | 3,4-Dichlorphenyl | | |
| 48 | $C(CH_3)(CH_2CH_3)$ | CHOH | 2-Chlor-4-fluorphenyl | | |
| 49 | $C(CH_3)(CH_2CH_3)$ | CHOH | 2-Trifluormethylphenyl | | |

Tabelle (Fortsetzung)

| Nr. | A | B | Ar | phys. Daten | IR, $cm^{-1}$ |
|---|---|---|---|---|---|
| 50 | $C(CH_3)(CH_2CH_3)$ | CHOH | 4-Trifluormethylphenyl | | |
| 51 | $C(CH_3)(CH_2CH_3)$ | CHOH | 4-Methoxyphenyl | | |
| 52 | $C(CH_3)(CH_2CH_3)$ | CHOH | 3,4-Dimethoxyphenyl | | |
| 53 | $C(CH_3)(CH_2CH_3)$ | CHOH | 4-Tetrafluorethoxyphenyl | | |
| 54 | $C(CH_3)(CH_2CH_3)$ | CHOH | 4-Trifluormethoxyphenyl | | |
| 55 | $C(CH_3)(CH_2CH_3)$ | CHOH | 2-Chlor-4-(4'-Chlorphenoxy)phenyl | | |
| 56 | $C(CH_3)(CH_2CH_3)$ | CHOH | 4-Phenoxyphenyl | | |
| 57 | $C(CH_3)(CH_2CH_3)$ | CHOH | 4-(4'-Chlorphenoxy)phenyl | | |
| 58 | $C(CH_3)(CH_2CH_3)$ | CHOH | 4-Benzyloxyphenyl | | |
| 59 | $C(CH_2CH_3)_2$ | CHOH | Phenyl | öl | 3203, 2963, 2935, 2879, 1452, 1424 |
| 60 | $C(CH_2CH_3)_2$ | CHOH | 2-Methylphenyl | öl | 3200, 2963, 1425, 1030, 758, 718 |
| 61 | $C(CH_2CH_3)_2$ | CHOH | 4-Methylphenyl | öl | 2965, 2936, 2879, 1478, 1455, 1424 |
| 62 | $C(CH_2CH_3)_2$ | CHOH | 2,4-Dimethylphenyl | | |
| 63 | $C(CH_2CH_3)_2$ | CHOH | 2,6-Dimethylphenyl | | |
| 64 | $C(CH_2CH_3)_2$ | CHOH | 2,4,6-Trimethylphenyl | | 161-164°C |
| 65 | $C(CH_2CH_3)_2$ | CHOH | 4-tert. Butylphenyl | | |
| 66 | $C(CH_2CH_3)_2$ | CHOH | 1-Naphthyl | | |
| 67 | $C(CH_2CH_3)_2$ | CHOH | 2-Naphthyl | | |
| 68 | $C(CH_2CH_3)_2$ | CHOH | 4-Biphenyl | | |
| 69 | $C(CH_2CH_3)_2$ | CHOH | 2-Fluorphenyl | | |
| 70 | $C(CH_2CH_3)_2$ | CHOH | 4-Fluorphenyl | öl | 3200, 2966, 2937, 2879, 1602, 1507 |
| 71 | $C(CH_2CH_3)_2$ | CHOH | 2,4-Difluorphenyl | | |
| 72 | $C(CH_2CH_3)_2$ | CHOH | 2-Chlorphenyl | | |
| 73 | $C(CH_2CH_3)_2$ | CHOH | 3-Chlorphenyl | | |
| 74 | $C(CH_2CH_3)_2$ | CHOH | 4-Chlorphenyl | öl | 3200, 2966, 2937, 2878, 1487, 1424 |

Tabelle (Fortsetzung)

| Nr. | A | B | Ar | phys. Daten IR, $cm^{-1}$ |
|---|---|---|---|---|
| 75 | $C(CH_2CH_3)_2$ | CHOH | 2,4-Dichlorphenyl | Fp.142-144°C |
| 76 | $C(CH_2CH_3)_2$ | CHOH | 3,4-Dichlorphenyl | |
| 77 | $C(CH_2CH_3)_2$ | CHOH | 2-Chlor-4-fluorphenyl | |
| 78 | $C(CH_2CH_3)_2$ | CHOH | 2-Trifluormethylphenyl | |
| 79 | $C(CH_2CH_3)_2$ | CHOH | 4-Trifluormethylphenyl | |
| 80 | $C(CH_2CH_3)_2$ | CHOH | 4-Methoxyphenyl | öl 3200,2963,1510,1246,1031,717 |
| 81 | $C(CH_2CH_3)_2$ | CHOH | 3,4-Dimethoxyphenyl | |
| 82 | $C(CH_2CH_3)_2$ | CHOH | 4-Tetrafluorethoxyphenyl | |
| 83 | $C(CH_2CH_3)_2$ | CHOH | 4-Trifluormethoxyphenyl | |
| 84 | $C(CH_2CH_3)_2$ | CHOH | 2-Chlor-4-(4'-Chlorphenoxy)phenyl | |
| 85 | $C(CH_2CH_3)_2$ | CHOH | 4-Phenoxyphenyl | |
| 86 | $C(CH_2CH_3)_2$ | CHOH | 4-(4'-Chlorphenoxy)phenyl | |
| 87 | $C(CH_2CH_3)_2$ | CHOH | 4-Benzyloxyphenyl | |
| 88 | Cyclopropylen | CHOH | Phenyl | öl 3231,3027,1424,1028,715,702 |
| 89 | Cyclopropylen | CHOH | 2-Methylphenyl | |
| 90 | Cyclopropylen | CHOH | 4-Methylphenyl | öl 3238,2921,1424,1044,820,716 |
| 91 | Cyclopropylen | CHOH | 2,4-Dimethylphenyl | |
| 92 | Cyclopropylen | CHOH | 2,6-Dimethylphenyl | |
| 93 | Cyclopropylen | CHOH | 2,4,6-Trimethylphenyl | |
| 94 | Cyclopropylen | CHOH | 4-tert. Butylphenyl | |
| 95 | Cyclopropylen | CHOH | 1-Naphthyl | |
| 96 | Cyclopropylen | CHOH | 2-Naphthyl | |
| 97 | Cyclopropylen | CHOH | 4-Biphenyl | |
| 98 | Cyclopropylen | CHOH | 2-Fluorphenyl | |
| 99 | Cyclopropylen | CHOH | 4-Fluorphenyl | öl 3200,2940,1508,1221,838 |

Tabelle (Fortsetzung)

| Nr. | A | B | Ar | phys. Daten | IR, $cm^{-1}$ |
|---|---|---|---|---|---|
| 100 | Cyclopropylen | CHOH | 2,4-Difluorphenyl | | |
| 101 | Cyclopropylen | CHOH | 2-Chlorphenyl | | |
| 102 | Cyclopropylen | CHOH | 3-Chlorphenyl | | |
| 103 | Cyclopropylen | CHOH | 4-Chlorphenyl | öl | 3200, 2924, 1425, 1014, 717 |
| 104 | Cyclopropylen | CHOH | 2,4-Dichlorphenyl | | |
| 105 | Cyclopropylen | CHOH | 3,4-Dichlorphenyl | | |
| 106 | Cyclopropylen | CHOH | 2-Chlor-4-fluorphenyl | | |
| 107 | Cyclopropylen | CHOH | 2-Trifluormethylphenyl | | |
| 108 | Cyclopropylen | CHOH | 4-Trifluormethylphenyl | | |
| 109 | Cyclopropylen | CHOH | 4-Methoxyphenyl | | |
| 110 | Cyclopropylen | CHOH | 3,4-Dimethoxyphenyl | | |
| 111 | Cyclopropylen | CHOH | 4-Tetrafluorethoxyphenyl | | |
| 112 | Cyclopropylen | CHOH | 4-Trifluormethoxyphenyl | | |
| 113 | Cyclopropylen | CHOH | 2-Chlor-4-(4'-Chlorphenoxy)phenyl | | |
| 114 | Cyclopropylen | CHOH | 4-Phenoxyphenyl | | |
| 115 | Cyclopropylen | CHOH | 4-(4'-Chlorphenoxy)phenyl | | |
| 116 | Cyclopropylen | CHOH | 4-Benzyloxyphenyl | | |
| 117 | Cyclopentylen | CHOH | Phenyl | öl | 3211, 2953, 2869, 1478, 1451, 1424 |
| 118 | Cyclopentylen | CHOH | 2-Methylphenyl | öl | 3225, 2954, 1442, 1031, 740, 719 |
| 119 | Cyclopentylen | CHOH | 4-Methylphenyl | öl | 3225, 2952, 2869, 1478, 1451, 1424 |
| 120 | Cyclopentylen | CHOH | 2,4-Dimethylphenyl | | |
| 121 | Cyclopentylen | CHOH | 2,6-Dimethylphenyl | | |
| 122 | Cyclopentylen | CHOH | 2,4,6-Trimethylphenyl | | 141-142°C |
| 123 | Cyclopentylen | CHOH | 4-tert. Butylphenyl | | |
| 124 | Cyclopentylen | CHOH | 1-Naphthyl | | |

Tabelle (Fortsetzung)

| Nr. | A | B | Ar | phys. Daten | IR, $cm^{-1}$ |
|---|---|---|---|---|---|
| 125 | Cyclopentylen | CHOH | 2-Naphthyl | | |
| 126 | Cyclopentylen | CHOH | 4-Biphenyl | | |
| 127 | Cyclopentylen | CHOH | 2-Fluorphenyl | | |
| 128 | Cyclopentylen | CHOH | 4-Fluorphenyl | öl | 3200,2954,2871,1603,1507,1425 |
| 129 | Cyclopentylen | CHOH | 2,4-Difluorphenyl | | |
| 130 | Cyclopentylen | CHOH | 2-Chlorphenyl | | |
| 131 | Cyclopentylen | CHOH | 3-Chlorphenyl | | |
| 132 | Cyclopentylen | CHOH | 4-Chlorphenyl | öl | 2954,2870,1484,1090,1046,1030 |
| 133 | Cyclopentylen | CHOH | 2,4-Dichlorphenyl | Fp.150-151°C | 3270,2952,2872,2706,2625,1541 |
| 134 | Cyclopentylen | CHOH | 3,4-Dichlorphenyl | | |
| 135 | Cyclopentylen | CHOH | 2-Chlor-4-fluorphenyl | | |
| 136 | Cyclopentylen | CHOH | 2-Trifluormethylphenyl | | |
| 137 | Cyclopentylen | CHOH | 4-Trifluormethylphenyl | | |
| 138 | Cyclopentylen | CHOH | 4-Methoxyphenyl | | 91-93°C |
| 139 | Cyclopentylen | CHOH | 3,4-Dimethoxyphenyl | | |
| 140 | Cyclopentylen | CHOH | 4-Tetrafluorethoxyphenyl | | |
| 141 | Cyclopentylen | CHOH | 4-Trifluormethoxyphenyl | | |
| 142 | Cyclopentylen | CHOH | 2-Chlor-4-(4'-Chlorphenoxy)phenyl | | |
| 143 | Cyclopentylen | CHOH | 4-Phenoxyphenyl | | |
| 144 | Cyclopentylen | CHOH | 4-(4'-Chlorphenoxy)phenyl | | |
| 145 | Cyclopentylen | CHOH | 4-Benzyloxyphenyl | | |
| 146 | Cyclohexylen | CHOH | Phenyl | öl | 2933,2860,1454,1424,1067,1042 |
| 147 | Cyclohexylen | CHOH | 2-Methylphenyl | öl | 3200,2934,1458,1069,737,719 |
| 148 | Cyclohexylen | CHOH | 4-Methylphenyl | öl | 2933,2860,1456,1423,1069,1042 |
| 149 | Cyclohexylen | CHOH | 2,4-Dimethylphenyl | | |

Tabelle (Fortsetzung)

| Nr. | A | B | Ar | phys. Daten IR, $cm^{-1}$ |
|---|---|---|---|---|
| 150 | Cyclohexylen | CHOH | 2,6-Dimethylphenyl | |
| 151 | Cyclohexylen | CHOH | 2,4,6-Trimethylphenyl | 164°C |
| 152 | Cyclohexylen | CHOH | 4-tert. Butylphenyl | 167-169°C |
| 153 | Cyclohexylen | CHOH | 1-Naphthyl | |
| 154 | Cyclohexylen | CHOH | 2-Naphthyl | |
| 155 | Cyclohexylen | CHOH | 4-Biphenyl | |
| 156 | Cyclohexylen | CHOH | 2-Fluorphenyl | |
| 157 | Cyclohexylen | CHOH | 4-Fluorphenyl | Fp.133-135°C<br>3264,2928,2862,1507,1453,1428,1222 |
| 158 | Cyclohexylen | CHOH | 2,4-Difluorphenyl | |
| 159 | Cyclohexylen | CHOH | 2-Chlorphenyl | |
| 160 | Cyclohexylen | CHOH | 3-Chlorphenyl | |
| 161 | Cyclohexylen | CHOH | 4-Chlorphenyl | öl 3152,2931,1480,1427,1076,717 |
| 162 | Cyclohexylen | CHOH | 2,4-Dichlorphenyl | 140-141°C |
| 163 | Cyclohexylen | CHOH | 3,4-Dichlorphenyl | |
| 164 | Cyclohexylen | CHOH | 2-Chlor-4-fluorphenyl | |
| 165 | Cyclohexylen | CHOH | 2-Trifluormethylphenyl | |
| 166 | Cyclohexylen | CHOH | 4-Trifluormethylphenyl | |
| 167 | Cyclohexylen | CHOH | 4-Methoxyphenyl | |
| 168 | Cyclohexylen | CHOH | 3,4-Dimethoxyphenyl | 126-129°C |
| 169 | Cyclohexylen | CHOH | 4-Tetrafluorethoxyphenyl | |
| 170 | Cyclohexylen | CHOH | 4-Trifluormethoxyphenyl | |
| 171 | Cyclohexylen | CHOH | 2-Chlor-4-(4'-Chlorphenoxy)phenyl | |
| 172 | Cyclohexylen | CHOH | 4-Phenoxyphenyl | |
| 173 | Cyclohexylen | CHOH | 4-(4'-Chlorphenoxy)phenyl | |

Tabelle (Fortsetzung)

| Nr. | A | B | Ar | phys. Daten | IR, $cm^{-1}$ |
|---|---|---|---|---|---|
| 174 | Cyclohexylen | CHOH | 4-Benzyloxyphenyl | | |
| 175 | $C(CH_3)(C_3H_7)$ | CHOH | Phenyl | öl | 2958,2933,2871,1452,1424,1068 |
| 176 | $C(CH_3)(C_3H_7)$ | CHOH | 2-Methylphenyl | | |
| 177 | $C(CH_3)(C_3H_7)$ | CHOH | 4-Methylphenyl | öl | 2958,2933,2870,1467,1456,1424 |
| 178 | $C(CH_3)(C_3H_7)$ | CHOH | 2,4-Dimethylphenyl | | |
| 179 | $C(CH_3)(C_3H_7)$ | CHOH | 2,6-Dimethylphenyl | | |
| 180 | $C(CH_3)(C_3H_7)$ | CHOH | 2,4,6-Trimethylphenyl | | |
| 181 | $C(CH_3)(C_3H_7)$ | CHOH | 4-tert. Butylphenyl | | |
| 182 | $C(CH_3)(C_3H_7)$ | CHOH | 1-Naphthyl | | |
| 183 | $C(CH_3)(C_3H_7)$ | CHOH | 2-Naphthyl | | |
| 184 | $C(CH_3)(C_3H_7)$ | CHOH | 4-Biphenyl | | |
| 185 | $C(CH_3)(C_3H_7)$ | CHOH | 2-Fluorphenyl | | |
| 186 | $C(CH_3)(C_3H_7)$ | CHOH | 4-Fluorphenyl | öl | 2959,2934,2871,1508,1425,1223 |
| 187 | $C(CH_3)(C_3H_7)$ | CHOH | 2,4-Difluorphenyl | | |
| 188 | $C(CH_3)(C_3H_7)$ | CHOH | 2-Chlorphenyl | | |
| 189 | $C(CH_3)(C_3H_7)$ | CHOH | 3-Chlorphenyl | | |
| 190 | $C(CH_3)(C_3H_7)$ | CHOH | 4-Chlorphenyl | öl | 2958,2933,2871,1486,1424,1090 |
| 191 | $C(CH_3)(C_3H_7)$ | CHOH | 2,4-Dichlorphenyl | | |
| 192 | $C(CH_3)(C_3H_7)$ | CHOH | 3,4-Dichlorphenyl | | |
| 193 | $C(CH_3)(C_3H_7)$ | CHOH | 2-Chlor-4-fluorphenyl | | |
| 194 | $C(CH_3)(C_3H_7)$ | CHOH | 2-Trifluormethylphenyl | | |
| 195 | $C(CH_3)(C_3H_7)$ | CHOH | 4-Trifluormethylphenyl | | |
| 196 | $C(CH_3)(C_3H_7)$ | CHOH | 4-Methoxyphenyl | | |
| 197 | $C(CH_3)(C_3H_7)$ | CHOH | 3,4-Dimethoxyphenyl | | |
| 198 | $C(CH_3)(C_3H_7)$ | CHOH | 4-Tetrafluorethoxyphenyl | | |

Tabelle (Fortsetzung)

| Nr. | A | B | Ar | phys. Daten | IR, $cm^{-1}$ |
|---|---|---|---|---|---|
| 199 | $C(CH_3)(C_3H_7)$ | CHOH | 4-Trifluormethoxyphenyl | | |
| 200 | $C(CH_3)(C_3H_7)$ | CHOH | 2-Chlor-4-(4'-Chlorphenoxy)phenyl | | |
| 201 | $C(CH_3)(C_3H_7)$ | CHOH | 4-Phenoxyphenyl | | |
| 202 | $C(CH_3)(C_3H_7)$ | CHOH | 4-(4'-Chlorphenoxy)phenyl | | |
| 203 | $C(CH_3)(C_3H_7)$ | CHOH | 4-Benzyloxyphenyl | | |
| 204 | $C(C_2H_5)(n-C_4H_9)$ | CHOH | Phenyl | Öl | 3196, 2956, 2934, 2869, 1452, 1424 |
| 205 | $C(C_2H_5)(n-C_4H_9)$ | CHOH | 2-Methylphenyl | | |
| 206 | $C(C_2H_5)(n-C_4H_9)$ | CHOH | 4-Methylphenyl | Öl | 3200, 2956, 2933, 2870, 1478, 1457 |
| 207 | $C(C_2H_5)(n-C_4H_9)$ | CHOH | 2,4-Dimethylphenyl | | |
| 208 | $C(C_2H_5)(n-C_4H_9)$ | CHOH | 2,6-Dimethylphenyl | | |
| 209 | $C(C_2H_5)(n-C_4H_9)$ | CHOH | 2,4,6-Trimethylphenyl | | |
| 210 | $C(C_2H_5)(n-C_4H_9)$ | CHOH | 4-tert. Butylphenyl | | |
| 211 | $C(C_2H_5)(n-C_4H_9)$ | CHOH | 1-Naphthyl | | |
| 212 | $C(C_2H_5)(n-C_4H_9)$ | CHOH | 2-Naphthyl | | |
| 213 | $C(C_2H_5)(n-C_4H_9)$ | CHOH | 4-Biphenyl | | |
| 214 | $C(C_2H_5)(n-C_4H_9)$ | CHOH | 2-Fluorphenyl | | |
| 215 | $C(C_2H_5)(n-C_4H_9)$ | CHOH | 4-Fluorphenyl | Öl | 3198, 2957, 2935, 2870, 1603, 1507 |
| 216 | $C(C_2H_5)(n-C_4H_9)$ | CHOH | 2,4-Difluorphenyl | | |
| 217 | $C(C_2H_5)(n-C_4H_9)$ | CHOH | 2-Chlorphenyl | | |
| 218 | $C(C_2H_5)(n-C_4H_9)$ | CHOH | 3-Chlorphenyl | | |
| 219 | $C(C_2H_5)(n-C_4H_9)$ | CHOH | 4-Chlorphenyl | Öl | 2957, 2934, 2870, 1487, 1458, 1424 |
| 220 | $C(C_2H_5)(n-C_4H_9)$ | CHOH | 2,4-Dichlorphenyl | | |
| 221 | $C(C_2H_5)(n-C_4H_9)$ | CHOH | 3,4-Dichlorphenyl | | |
| 222 | $C(C_2H_5)(n-C_4H_9)$ | CHOH | 2-Chlor-4-fluorphenyl | | |
| 223 | $C(C_2H_5)(n-C_4H_9)$ | CHOH | 2-Trifluormethylphenyl | | |

Tabelle (Fortsetzung)

| Nr. | A | B | Ar | phys. Daten IR, $cm^{-1}$ |
|---|---|---|---|---|
| 224 | $C(C_2H_5)(n-C_4H_9)$ | CHOH | 4-Trifluormethylphenyl | |
| 225 | $C(C_2H_5)(n-C_4H_9)$ | CHOH | 4-Methoxyphenyl | |
| 226 | $C(C_2H_5)(n-C_4H_9)$ | CHOH | 3,4-Dimethoxyphenyl | |
| 227 | $C(C_2H_5)(n-C_4H_9)$ | CHOH | 4-Tetrafluorethoxyphenyl | |
| 228 | $C(C_2H_5)(n-C_4H_9)$ | CHOH | 4-Trifluormethoxyphenyl | |
| 229 | $C(C_2H_5)(n-C_4H_9)$ | CHOH | 2-Chlor-4-(4'-Chlorphenoxy)-phenyl | |
| 230 | $C(C_2H_5)(n-C_4H_9)$ | CHOH | 4-Phenoxyphenyl | |
| 231 | $C(C_2H_5)(n-C_4H_9)$ | CHOH | 4-(4'-Chlorphenoxy)phenyl | |
| 232 | $C(C_2H_5)(n-C_4H_9)$ | CHOH | 4-Benzyloxyphenyl | |
| 233 | $CH(tert.C_4H_9)$ | CHOH | Phenyl | |
| 234 | $CH(tert.C_4H_9)$ | CHOH | 2-Methylphenyl | |
| 235 | $CH(tert.C_4H_9)$ | CHOH | 4-Methylphenyl | |
| 236 | $CH(tert.C_4H_9)$ | CHOH | 2,4-Dimethylphenyl | |
| 237 | $CH(tert.C_4H_9)$ | CHOH | 2,6-Dimethylphenyl | |
| 238 | $CH(tert.C_4H_9)$ | CHOH | 2,4,6-Trimethylphenyl | |
| 239 | $CH(tert.C_4H_9)$ | CHOH | 4-tert. Butylphenyl | |
| 240 | $CH(tert.C_4H_9)$ | CHOH | 1-Naphthyl | |
| 241 | $CH(tert.C_4H_9)$ | CHOH | 2-Naphthyl | |
| 242 | $CH(tert.C_4H_9)$ | CHOH | 4-Biphenyl | |
| 243 | $CH(tert.C_4H_9)$ | CHOH | 2-Fluorphenyl | |
| 244 | $CH(tert.C_4H_9)$ | CHOH | 4-Fluorphenyl | Öl 2.90, 2.75 (dd, 2H, $-CH_2-Py$) |
| 245 | $CH(tert.C_4H_9)$ | CHOH | 2,4-Difluorphenyl | |
| 246 | $CH(tert.C_4H_9)$ | CHOH | 2-Chlorphenyl | |
| 247 | $CH(tert.C_4H_9)$ | CHOH | 3-Chlorphenyl | |
| 248 | $CH(tert.C_4H_9)$ | CHOH | 4-Chlorphenyl | |

Tabelle (Fortsetzung)

| Nr. | A | B | Ar | phys. Daten | IR, $cm^{-1}$ |
|---|---|---|---|---|---|
| 249 | $CH(tert.C_4H_9)$ | CHOH | 2,4-Dichlorphenyl | | |
| 250 | $CH(tert.C_4H_9)$ | CHOH | 3,4-Dichlorphenyl | | |
| 251 | $CH(tert.C_4H_9)$ | CHOH | 2-Chlor-4-fluorphenyl | | |
| 252 | $CH(tert.C_4H_9)$ | CHOH | 2-Trifluormethylphenyl | | |
| 253 | $CH(tert.C_4H_9)$ | CHOH | 4-Trifluormethylphenyl | | |
| 254 | $CH(tert.C_4H_9)$ | CHOH | 4-Methoxyphenyl | | |
| 255 | $CH(tert.C_4H_9)$ | CHOH | 3,4-Dimethoxyphenyl | | |
| 256 | $CH(tert.C_4H_9)$ | CHOH | 4-Tetrafluorethoxyphenyl | | |
| 257 | $CH(tert.C_4H_9)$ | CHOH | 4-Trifluormethoxyphenyl | | |
| 258 | $CH(tert.C_4H_9)$ | CHOH | 2-Chlor-4-(4'-Chlorphenoxy)phenyl | | |
| 259 | $CH(tert.C_4H_9)$ | CHOH | 4-Phenoxyphenyl | | |
| 260 | $CH(tert.C_4H_9)$ | CHOH | 4-(4'-Chlorphenoxy)phenyl | | |
| 261 | $CH(tert.C_4H_9)$ | CHOH | 4-Benzyloxyphenyl | | |
| 262 | $C(CH_3)_2$ | C=O | Phenyl | | |
| 263 | $C(CH_3)_2$ | C=O | 2-Methylphenyl | | |
| 264 | $C(CH_3)_2$ | C=O | 4-Methylphenyl | öl | 3.05 (s,2H,$-CH_2$-Py) |
| 265 | $C(CH_3)_2$ | C=O | 2,4-Dimethylphenyl | | |
| 266 | $C(CH_3)_2$ | C=O | 2,6-Dimethylphenyl | | |
| 267 | $C(CH_3)_2$ | C=O | 2,4,6-Trimethylphenyl | | |
| 268 | $C(CH_3)_2$ | C=O | 4-tert. Butylphenyl | | |
| 269 | $C(CH_3)_2$ | C=O | 1-Naphthyl | | |
| 270 | $C(CH_3)_2$ | C=O | 2-Naphthyl | | |
| 271 | $C(CH_3)_2$ | C=O | 4-Biphenyl | | |
| 272 | $C(CH_3)_2$ | C=O | 2-Fluorphenyl | | |
| 273 | $C(CH_3)_2$ | C=O | 4-Fluorphenyl | öl | 2932,1670,1588,1479,1453,1423 |

Tabelle (Fortsetzung)

| Nr. | A | B | Ar | phys. Daten IR, $cm^{-1}$ |
|---|---|---|---|---|
| 274 | $C(CH_3)_2$ | C=O | 2,4-Difluorphenyl | |
| 275 | $C(CH_3)_2$ | C=O | 2-Chlorphenyl | |
| 276 | $C(CH_3)_2$ | C=O | 3-Chlorphenyl | |
| 277 | $C(CH_3)_2$ | C=O | 4-Chlorphenyl | öl 3.05 (s, 2H, $-CH_2-Py$) |
| 278 | $C(CH_3)_2$ | C=O | 2,4-Dichlorphenyl | |
| 279 | $C(CH_3)_2$ | C=O | 3,4-Dichlorphenyl | |
| 280 | $C(CH_3)_2$ | C=O | 2-Chlor-4-fluorphenyl | |
| 281 | $C(CH_3)_2$ | C=O | 2-Trifluormethylphenyl | |
| 282 | $C(CH_3)_2$ | C=O | 4-Trifluormethylphenyl | |
| 283 | $C(CH_3)_2$ | C=O | 4-Methoxyphenyl | |
| 284 | $C(CH_3)_2$ | C=O | 3,4-Dimethoxyphenyl | |
| 285 | $C(CH_3)_2$ | C=O | 4-Tetrafluorethoxyphenyl | |
| 286 | $C(CH_3)_2$ | C=O | 4-Trifluormethoxyphenyl | |
| 287 | $C(CH_3)_2$ | C=O | 2-Chlor-4-(4'-Chlorphenoxy)phenyl | |
| 288 | $C(CH_3)_2$ | C=O | 4-Phenoxyphenyl | |
| 289 | $C(CH_3)_2$ | C=O | 4-(4'-Chlorphenoxy)phenyl | |
| 290 | $C(CH_3)_2$ | C=O | 4-Benzyloxyphenyl | |
| 291 | $C(CH_3)(CH_2CH_3)$ | C=O | Phenyl | |
| 292 | $C(CH_3)(CH_2CH_3)$ | C=O | 2-Methylphenyl | |
| 293 | $C(CH_3)(CH_2CH_3)$ | C=O | 4-Methylphenyl | |
| 294 | $C(CH_3)(CH_2CH_3)$ | C=O | 2,4-Dimethylphenyl | |
| 295 | $C(CH_3)(CH_2CH_3)$ | C=O | 2,6-Dimethylphenyl | |
| 296 | $C(CH_3)(CH_2CH_3)$ | C=O | 2,4,6-Trimethylphenyl | |
| 297 | $C(CH_3)(CH_2CH_3)$ | C=O | 4-tert. Butylphenyl | |
| 298 | $C(CH_3)(CH_2CH_3)$ | C=O | 1-Naphthyl | |

Tabelle (Fortsetzung)

| Nr. | A | B | Ar | phys. Daten IR, $cm^{-1}$ |
|---|---|---|---|---|
| 299 | $C(CH_3)(CH_2CH_3)$ | C=O | 2-Naphthyl | |
| 300 | $C(CH_3)(CH_2CH_3)$ | C=O | 4-Biphenyl | |
| 301 | $C(CH_3)(CH_2CH_3)$ | C=O | 2-Fluorphenyl | |
| 302 | $C(CH_3)(CH_2CH_3)$ | C=O | 4-Fluorphenyl | |
| 303 | $C(CH_3)(CH_2CH_3)$ | C=O | 2,4-Difluorphenyl | |
| 304 | $C(CH_3)(CH_2CH_3)$ | C=O | 2-Chlorphenyl | |
| 305 | $C(CH_3)(CH_2CH_3)$ | C=O | 3-Chlorphenyl | |
| 306 | $C(CH_3)(CH_2CH_3)$ | C=O | 4-Chlorphenyl | |
| 307 | $C(CH_3)(CH_2CH_3)$ | C=O | 2,4-Dichlorphenyl | |
| 308 | $C(CH_3)(CH_2CH_3)$ | C=O | 3,4-Dichlorphenyl | |
| 309 | $C(CH_3)(CH_2CH_3)$ | C=O | 2-Chlor-4-fluorphenyl | |
| 310 | $C(CH_3)(CH_2CH_3)$ | C=O | 2-Trifluormethylphenyl | |
| 311 | $C(CH_3)(CH_2CH_3)$ | C=O | 4-Trifluormethylphenyl | |
| 312 | $C(CH_3)(CH_2CH_3)$ | C=O | 4-Methoxyphenyl | |
| 313 | $C(CH_3)(CH_2CH_3)$ | C=O | 3,4-Dimethoxyphenyl | |
| 314 | $C(CH_3)(CH_2CH_3)$ | C=O | 4-Tetrafluorethoxyphenyl | |
| 315 | $C(CH_3)(CH_2CH_3)$ | C=O | 4-Trifluormethoxyphenyl | |
| 316 | $C(CH_3)(CH_2CH_3)$ | C=O | 2-Chlor-4-(4'-Chlorphenoxy)phenyl | |
| 317 | $C(CH_3)(CH_2CH_3)$ | C=O | 4-Phenoxyphenyl | |
| 318 | $C(CH_3)(CH_2CH_3)$ | C=O | 4-(4'-Chlorphenoxy)phenyl | |
| 319 | $C(CH_3)(CH_2CH_3)$ | C=O | 4-Benzyloxyphenyl | |
| 320 | $C(CH_2CH_3)_2$ | C=O | Phenyl | |
| 321 | $C(CH_2CH_3)_2$ | C=O | 2-Methylphenyl | |
| 322 | $C(CH_2CH_3)_2$ | C=O | 4-Methylphenyl | |
| 323 | $C(CH_2CH_3)_2$ | C=O | 2,4-Dimethylphenyl | |

Tabelle (Fortsetzung)

| Nr. | A | B | Ar | phys. Daten | IR, $cm^{-1}$ |
|---|---|---|---|---|---|
| 324 | $C(CH_2CH_3)_2$ | C=O | 2,6-Dimethylphenyl | | |
| 325 | $C(CH_2CH_3)_2$ | C=O | 2,4,6-Trimethylphenyl | | |
| 326 | $C(CH_2CH_3)_2$ | C=O | 4-tert. Butylphenyl | | |
| 327 | $C(CH_2CH_3)_2$ | C=O | 1-Naphthyl | | |
| 328 | $C(CH_2CH_3)_2$ | C=O | 2-Naphthyl | | |
| 329 | $C(CH_2CH_3)_2$ | C=O | 4-Biphenyl | | |
| 330 | $C(CH_2CH_3)_2$ | C=O | 2-Fluorphenyl | | |
| 331 | $C(CH_2CH_3)_2$ | C=O | 4-Fluorphenyl | öl | 3.08 (s,2H,$-CH_2-$Py) |
| 332 | $C(CH_2CH_3)_2$ | C=O | 2,4-Difluorphenyl | | |
| 333 | $C(CH_2CH_3)_2$ | C=O | 2-Chlorphenyl | | |
| 334 | $C(CH_2CH_3)_2$ | C=O | 3-Chlorphenyl | | |
| 335 | $C(CH_2CH_3)_2$ | C=O | 4-Chlorphenyl | | |
| 336 | $C(CH_2CH_3)_2$ | C=O | 2,4-Dichlorphenyl | | |
| 337 | $C(CH_2CH_3)_2$ | C=O | 3,4-Dichlorphenyl | | |
| 338 | $C(CH_2CH_3)_2$ | C=O | 2-Chlor-4-fluorphenyl | | |
| 339 | $C(CH_2CH_3)_2$ | C=O | 2-Trifluormethylphenyl | | |
| 340 | $C(CH_2CH_3)_2$ | C=O | 4-Trifluormethylphenyl | | |
| 341 | $C(CH_2CH_3)_2$ | C=O | 4-Methoxyphenyl | | |
| 342 | $C(CH_2CH_3)_2$ | C=O | 3,4-Dimethoxyphenyl | | |
| 343 | $C(CH_2CH_3)_2$ | C=O | 4-Tetrafluorethoxyphenyl | | |
| 344 | $C(CH_2CH_3)_2$ | C=O | 4-Trifluormethoxyphenyl | | |
| 345 | $C(CH_2CH_3)_2$ | C=O | 2-Chlor-4-(4'-Chlorphenoxy)phenyl | | |
| 346 | $C(CH_2CH_3)_2$ | C=O | 4-Phenoxyphenyl | | |
| 347 | $C(CH_2CH_3)_2$ | C=O | 4-(4'-Chlorphenoxy)phenyl | | |
| 348 | $C(CH_2CH_3)_2$ | C=O | 4-Benzyloxyphenyl | | |

Tabelle (Fortsetzung)

| Nr. | A | B | Ar | phys. Daten | IR, $cm^{-1}$ |
|---|---|---|---|---|---|
| 349 | Cyclopropylen | C=O | Phenyl | | |
| 350 | Cyclopropylen | C=O | 2-Methylphenyl | | |
| 351 | Cyclopropylen | C=O | 4-Methylphenyl | öl | 1671,1808,1423,1177,831,715 |
| 352 | Cyclopropylen | C=O | 2,4-Dimethylphenyl | | |
| 353 | Cyclopropylen | C=O | 2,6-Dimethylphenyl | | |
| 354 | Cyclopropylen | C=O | 2,4,6-Trimethylphenyl | | |
| 355 | Cyclopropylen | C=O | 4-tert. Butylphenyl | | |
| 356 | Cyclopropylen | C=O | 1-Naphthyl | | |
| 357 | Cyclopropylen | C=O | 2-Naphthyl | | |
| 358 | Cyclopropylen | C=O | 4-Biphenyl | | |
| 359 | Cyclopropylen | C=O | 2-Fluorphenyl | | |
| 360 | Cyclopropylen | C=O | 4-Fluorphenyl | öl | 1676,1600,1505,1229,848,715 |
| 361 | Cyclopropylen | C=O | 2,4-Difluorphenyl | | |
| 362 | Cyclopropylen | C=O | 2-Chlorphenyl | | |
| 363 | Cyclopropylen | C=O | 3-Chlorphenyl | | |
| 364 | Cyclopropylen | C=O | 4-Chlorphenyl | öl | 1675,1589,1424,1051,841,715 |
| 365 | Cyclopropylen | C=O | 2,4-Dichlorphenyl | | |
| 366 | Cyclopropylen | C=O | 3,4-Dichlorphenyl | | |
| 367 | Cyclopropylen | C=O | 2-Chlor-4-fluorphenyl | | |
| 368 | Cyclopropylen | C=O | 2-Trifluormethylphenyl | | |
| 369 | Cyclopropylen | C=O | 4-Trifluormethylphenyl | | |
| 370 | Cyclopropylen | C=O | 4-Methoxyphenyl | | |
| 371 | Cyclopropylen | C=O | 3,4-Dimethoxyphenyl | | |
| 372 | Cyclopropylen | C=O | 4-Tetrafluorethoxyphenyl | | |
| 373 | Cyclopropylen | C=O | 4-Trifluormethoxyphenyl | | |

Tabelle (Fortsetzung)

| Nr. | A | B | Ar | phys. Daten | IR, $cm^{-1}$ |
|---|---|---|---|---|---|
| 374 | Cyclopropylen | C=O | 2-Chlor-4-(4'-Chlorphenoxy)phenyl | | |
| 375 | Cyclopropylen | C=O | 4-Phenoxyphenyl | | |
| 376 | Cyclopropylen | C=O | 4-(4'-Chlorphenoxy)phenyl | | |
| 377 | Cyclopropylen | C=O | 4-Benzyloxyphenyl | | |
| 378 | Cyclopentylen | C=O | Phenyl | | |
| 379 | Cyclopentylen | C=O | 2-Methylphenyl | | |
| 380 | Cyclopentylen | C=O | 4-Methylphenyl | | |
| 381 | Cyclopentylen | C=O | 2,4-Dimethylphenyl | | |
| 382 | Cyclopentylen | C=O | 2,6-Dimethylphenyl | | |
| 383 | Cyclopentylen | C=O | 2,4,6-Trimethylphenyl | | |
| 384 | Cyclopentylen | C=O | 4-tert. Butylphenyl | | |
| 385 | Cyclopentylen | C=O | 1-Naphthyl | | |
| 386 | Cyclopentylen | C=O | 2-Naphthyl | | |
| 387 | Cyclopentylen | C=O | 4-Biphenyl | | |
| 388 | Cyclopentylen | C=O | 2-Fluorphenyl | | |
| 389 | Cyclopentylen | C=O | 4-Fluorphenyl | öl | 3.15 (s,2H,$-CH_2$-Py) |
| 390 | Cyclopentylen | C=O | 2,4-Difluorphenyl | | |
| 391 | Cyclopentylen | C=O | 2-Chlorphenyl | | |
| 392 | Cyclopentylen | C=O | 3-Chlorphenyl | | |
| 393 | Cyclopentylen | C=O | 4-Chlorphenyl | öl | 3.15 (s,2H,$-CH_2$-Py) |
| 394 | Cyclopentylen | C=O | 2,4-Dichlorphenyl | öl | 1691,1583,1423,1105,825,717 |
| 395 | Cyclopentylen | C=O | 3,4-Dichlorphenyl | | |
| 396 | Cyclopentylen | C=O | 2-Chlor-4-fluorphenyl | | |
| 397 | Cyclopentylen | C=O | 2-Trifluormethylphenyl | | |
| 398 | Cyclopentylen | C=O | 4-Trifluormethylphenyl | | |

Tabelle (Fortsetzung)

| Nr. | A | B | Ar | phys. Daten IR, $cm^{-1}$ |
|---|---|---|---|---|
| 399 | Cyclopentylen | C=O | 4-Methoxyphenyl | |
| 400 | Cyclopentylen | C=O | 3,4-Dimethoxyphenyl | |
| 401 | Cyclopentylen | C=O | 4-Tetrafluorethoxyphenyl | |
| 402 | Cyclopentylen | C=O | 4-Trifluormethoxyphenyl | |
| 403 | Cyclopentylen | C=O | 2-Chlor-4-(4'-Chlorphenoxy)phenyl | |
| 404 | Cyclopentylen | C=O | 4-Phenoxyphenyl | |
| 405 | Cyclopentylen | C=O | 4-(4'-Chlorphenoxy)phenyl | |
| 406 | Cyclopentylen | C=O | 4-Benzyloxyphenyl | |
| 407 | Cyclohexylen | C=O | Phenyl | |
| 408 | Cyclohexylen | C=O | 2-Methylphenyl | |
| 409 | Cyclohexylen | C=O | 4-Methylphenyl | |
| 410 | Cyclohexylen | C=O | 2,4-Dimethylphenyl | |
| 411 | Cyclohexylen | C=O | 2,6-Dimethylphenyl | |
| 412 | Cyclohexylen | C=O | 2,4,6-Trimethylphenyl | |
| 413 | Cyclohexylen | C=O | 4-tert. Butylphenyl | |
| 414 | Cyclohexylen | C=O | 1-Naphthyl | |
| 415 | Cyclohexylen | C=O | 2-Naphthyl | |
| 416 | Cyclohexylen | C=O | 4-Biphenyl | |
| 417 | Cyclohexylen | C=O | 2-Fluorphenyl | |
| 418 | Cyclohexylen | C=O | 4-Fluorphenyl | |
| 419 | Cyclohexylen | C=O | 2,4-Difluorphenyl | |
| 420 | Cyclohexylen | C=O | 2-Chlorphenyl | |
| 421 | Cyclohexylen | C=O | 3-Chlorphenyl | |
| 422 | Cyclohexylen | C=O | 4-Chlorphenyl | Öl 3.1 (s, 2H, $-CH_2$-Py) |
| 423 | Cyclohexylen | C=O | 2,4-Dichlorphenyl | |

Tabelle (Fortsetzung)

| Nr. | A | B | Ar | phys. Daten IR, $cm^{-1}$ |
|---|---|---|---|---|
| 424 | Cyclohexylen | C=O | 3,4-Dichlorphenyl | |
| 425 | Cyclohexylen | C=O | 2-Chlor-4-fluorphenyl | |
| 426 | Cyclohexylen | C=O | 2-Trifluormethylphenyl | |
| 427 | Cyclohexylen | C=O | 4-Trifluormethylphenyl | |
| 428 | Cyclohexylen | C=O | 4-Methoxyphenyl | |
| 429 | Cyclohexylen | C=O | 3,4-Dimethoxyphenyl | |
| 430 | Cyclohexylen | C=O | 4-Tetrafluorethoxyphenyl | |
| 431 | Cyclohexylen | C=O | 4-Trifluormethoxyphenyl | |
| 432 | Cyclohexylen | C=O | 2-Chlor-4-(4'-Chlorphenoxy)phenyl | |
| 433 | Cyclohexylen | C=O | 4-Phenoxyphenyl | |
| 434 | Cyclohexylen | C=O | 4-(4'-Chlorphenoxy)phenyl | |
| 435 | Cyclohexylen | C=O | 4-Benzyloxyphenyl | |
| 436 | $C(CH_3)(C_3H_7)$ | C=O | Phenyl | |
| 437 | $C(CH_3)(C_3H_7)$ | C=O | 2-Methylphenyl | |
| 438 | $C(CH_3)(C_3H_7)$ | C=O | 4-Methylphenyl | |
| 439 | $C(CH_3)(C_3H_7)$ | C=O | 2,4-Dimethylphenyl | |
| 440 | $C(CH_3)(C_3H_7)$ | C=O | 2,6-Dimethylphenyl | |
| 441 | $C(CH_3)(C_3H_7)$ | C=O | 2,4,6-Trimethylphenyl | |
| 442 | $C(CH_3)(C_3H_7)$ | C=O | 4-tert. Butylphenyl | |
| 443 | $C(CH_3)(C_3H_7)$ | C=O | 1-Naphthyl | |
| 444 | $C(CH_3)(C_3H_7)$ | C=O | 2-Naphthyl | |
| 445 | $C(CH_3)(C_3H_7)$ | C=O | 4-Biphenyl | |
| 446 | $C(CH_3)(C_3H_7)$ | C=O | 2-Fluorphenyl | |
| 447 | $C(CH_3)(C_3H_7)$ | C=O | 4-Fluorphenyl | |
| 448 | $C(CH_3)(C_3H_7)$ | C=O | 2,4-Difluorphenyl | |

Tabelle (Fortsetzung)

| Nr. | A | B | Ar | phys. Daten IR, $cm^{-1}$ |
|---|---|---|---|---|
| 449 | $C(CH_3)(C_3H_7)$ | C=O | 2-Chlorphenyl | |
| 450 | $C(CH_3)(C_3H_7)$ | C=O | 3-Chlorphenyl | |
| 451 | $C(CH_3)(C_3H_7)$ | C=O | 4-Chlorphenyl | |
| 452 | $C(CH_3)(C_3H_7)$ | C=O | 2,4-Dichlorphenyl | |
| 453 | $C(CH_3)(C_3H_7)$ | C=O | 3,4-Dichlorphenyl | |
| 454 | $C(CH_3)(C_3H_7)$ | C=O | 2-Chlor-4-fluorphenyl | |
| 455 | $C(CH_3)(C_3H_7)$ | C=O | 2-Trifluormethylphenyl | |
| 456 | $C(CH_3)(C_3H_7)$ | C=O | 4-Trifluormethylphenyl | |
| 457 | $C(CH_3)(C_3H_7)$ | C=O | 4-Methoxyphenyl | |
| 458 | $C(CH_3)(C_3H_7)$ | C=O | 3,4-Dimethoxyphenyl | |
| 459 | $C(CH_3)(C_3H_7)$ | C=O | 4-Tetrafluorethoxyphenyl | |
| 460 | $C(CH_3)(C_3H_7)$ | C=O | 4-Trifluormethoxyphenyl | |
| 461 | $C(CH_3)(C_3H_7)$ | C=O | 2-Chlor-4-(4'-Chlorphenoxy)phenyl | |
| 462 | $C(CH_3)(C_3H_7)$ | C=O | 4-Phenoxyphenyl | |
| 463 | $C(CH_3)(C_3H_7)$ | C=O | 4-(4'-Chlorphenoxy)phenyl | |
| 464 | $C(CH_3)(C_3H_7)$ | C=O | 4-Benzyloxyphenyl | |
| 465 | $C(C_2H_5)(n-C_4H_9)$ | C=O | Phenyl | |
| 466 | $C(C_2H_5)(n-C_4H_9)$ | C=O | 2-Methylphenyl | |
| 467 | $C(C_2H_5)(n-C_4H_9)$ | C=O | 4-Methylphenyl | |
| 468 | $C(C_2H_5)(n-C_4H_9)$ | C=O | 2,4-Dimethylphenyl | |
| 469 | $C(C_2H_5)(n-C_4H_9)$ | C=O | 2,6-Dimethylphenyl | |
| 470 | $C(C_2H_5)(n-C_4H_9)$ | C=O | 2,4,6-Trimethylphenyl | |
| 471 | $C(C_2H_5)(n-C_4H_9)$ | C=O | 4-tert. Butylphenyl | |
| 472 | $C(C_2H_5)(n-C_4H_9)$ | C=O | 1-Naphthyl | |
| 473 | $C(C_2H_5)(n-C_4H_9)$ | C=O | 2-Naphthyl | |

Tabelle (Fortsetzung)

| Nr. | A | B | Ar | phys. Daten IR, $cm^{-1}$ |
|---|---|---|---|---|
| 474 | $C(C_2H_5)(n-C_4H_9)$ | C=O | 4-Biphenyl | |
| 475 | $C(C_2H_5)(n-C_4H_9)$ | C=O | 2-Fluorphenyl | |
| 476 | $C(C_2H_5)(n-C_4H_9)$ | C=O | 4-Fluorphenyl | |
| 477 | $C(C_2H_5)(n-C_4H_9)$ | C=O | 2,4-Difluorphenyl | |
| 478 | $C(C_2H_5)(n-C_4H_9)$ | C=O | 2-Chlorphenyl | |
| 479 | $C(C_2H_5)(n-C_4H_9)$ | C=O | 3-Chlorphenyl | |
| 480 | $C(C_2H_5)(n-C_4H_9)$ | C=O | 4-Chlorphenyl | |
| 481 | $C(C_2H_5)(n-C_4H_9)$ | C=O | 2,4-Dichlorphenyl | |
| 482 | $C(C_2H_5)(n-C_4H_9)$ | C=O | 3,4-Dichlorphenyl | |
| 483 | $C(C_2H_5)(n-C_4H_9)$ | C=O | 2-Chlor-4-fluorphenyl | |
| 484 | $C(C_2H_5)(n-C_4H_9)$ | C=O | 2-Trifluormethylphenyl | |
| 485 | $C(C_2H_5)(n-C_4H_9)$ | C=O | 4-Trifluormethylphenyl | |
| 486 | $C(C_2H_5)(n-C_4H_9)$ | C=O | 4-Methoxyphenyl | |
| 487 | $C(C_2H_5)(n-C_4H_9)$ | C=O | 3,4-Dimethoxyphenyl | |
| 488 | $C(C_2H_5)(n-C_4H_9)$ | C=O | 4-Tetrafluorethoxyphenyl | |
| 489 | $C(C_2H_5)(n-C_4H_9)$ | C=O | 4-Trifluormethoxyphenyl | |
| 490 | $C(C_2H_5)(n-C_4H_9)$ | C=O | 2-Chlor-4-(4'-Chlorphenoxy)-phenyl | |
| 491 | $C(C_2H_5)(n-C_4H_9)$ | C=O | 4-Phenoxyphenyl | |
| 492 | $C(C_2H_5)(n-C_4H_9)$ | C=O | 4-(4'-Chlorphenoxy)phenyl | |
| 493 | $C(C_2H_5)(n-C_4H_9)$ | C=O | 4-Benzyloxyphenyl | |
| 494 | $CH(tert.C_4H_9)$ | C=O | Phenyl | |
| 495 | $CH(tert.C_4H_9)$ | C=O | 2-Methylphenyl | |
| 496 | $CH(tert.C_4H_9)$ | C=O | 4-Methylphenyl | |
| 497 | $CH(tert.C_4H_9)$ | C=O | 2,4-Dimethylphenyl | |
| 498 | $CH(tert.C_4H_9)$ | C=O | 2,6-Dimethylphenyl | |

Tabelle (Fortsetzung)

| Nr. | A | B | Ar | phys. Daten IR, $cm^{-1}$ |
|---|---|---|---|---|
| 499 | CH(tert.$C_4H_9$) | C=O | 2,4,6-Trimethylphenyl | |
| 500 | CH(tert.$C_4H_9$) | C=O | 4-tert. Butylphenyl | |
| 501 | CH(tert.$C_4H_9$) | C=O | 1-Naphthyl | |
| 502 | CH(tert.$C_4H_9$) | C=O | 2-Naphthyl | |
| 503 | CH(tert.$C_4H_9$) | C=O | 4-Biphenyl | |
| 504 | CH(tert.$C_4H_9$) | C=O | 2-Fluorphenyl | |
| 505 | CH(tert.$C_4H_9$) | C=O | 4-Fluorphenyl | |
| 506 | CH(tert.$C_4H_9$) | C=O | 2,4-Difluorphenyl | |
| 507 | CH(tert.$C_4H_9$) | C=O | 2-Chlorphenyl | |
| 508 | CH(tert.$C_4H_9$) | C=O | 3-Chlorphenyl | |
| 509 | CH(tert.$C_4H_9$) | C=O | 4-Chlorphenyl | |
| 510 | CH(tert.$C_4H_9$) | C=O | 2,4-Dichlorphenyl | |
| 511 | CH(tert.$C_4H_9$) | C=O | 3,4-Dichlorphenyl | |
| 512 | CH(tert.$C_4H_9$) | C=O | 2-Chlor-4-fluorphenyl | |
| 513 | CH(tert.$C_4H_9$) | C=O | 2-Trifluormethylphenyl | |
| 514 | CH(tert.$C_4H_9$) | C=O | 4-Trifluormethylphenyl | |
| 515 | CH(tert.$C_4H_9$) | C=O | 4-Methoxyphenyl | |
| 516 | CH(tert.$C_4H_9$) | C=O | 3,4-Dimethoxyphenyl | |
| 517 | CH(tert.$C_4H_9$) | C=O | 4-Tetrafluorethoxyphenyl | |
| 518 | CH(tert.$C_4H_9$) | C=O | 4-Trifluormethoxyphenyl | |
| 519 | CH(tert.$C_4H_9$) | C=O | 2-Chlor-4-(4'-Chlorphenoxy)phenyl | |
| 520 | CH(tert.$C_4H_9$) | C=O | 4-Phenoxyphenyl | |
| 521 | CH(tert.$C_4H_9$) | C=O | 4-(4'-Chlorphenoxy)phenyl | |
| 522 | CH(tert.$C_4H_9$) | C=O | 4-Benzyloxyphenyl | |
| 523 | $C(CH_3)_2$ | CHCl | Phenyl | 75-80°C |

Tabelle (Fortsetzung)

| Nr. | A | B | Ar | phys. Daten IR, $cm^{-1}$ |
|---|---|---|---|---|
| 524 | $C(CH_3)_2$ | CHCl | 2-Methylphenyl | 212-215°C |
| 525 | $C(CH_3)_2$ | CHCl | 4-Methylphenyl | 108-109°C |
| 526 | $C(CH_3)_2$ | CHCl | 2,4-Dimethylphenyl | |
| 527 | $C(CH_3)_2$ | CHCl | 2,6-Dimethylphenyl | |
| 528 | $C(CH_3)_2$ | CHCl | 2,4,6-Trimethylphenyl | 215-218°C |
| 529 | $C(CH_3)_2$ | CHCl | 4-tert. Butylphenyl | 143-147°C |
| 530 | $C(CH_3)_2$ | CHCl | 1-Naphthyl | |
| 531 | $C(CH_3)_2$ | CHCl | 2-Naphthyl | |
| 532 | $C(CH_3)_2$ | CHCl | 4-Biphenyl | |
| 533 | $C(CH_3)_2$ | CHCl | 2-Fluorphenyl | |
| 534 | $C(CH_3)_2$ | CHCl | 4-Fluorphenyl | |
| 535 | $C(CH_3)_2$ | CHCl | 2,4-Difluorphenyl | |
| 536 | $C(CH_3)_2$ | CHCl | 2-Chlorphenyl | |
| 537 | $C(CH_3)_2$ | CHCl | 3-Chlorphenyl | |
| 538 | $C(CH_3)_2$ | CHCl | 4-Chlorphenyl | 155-158°C |
| 539 | $C(CH_3)_2$ | CHCl | 2,4-Dichlorphenyl | |
| 540 | $C(CH_3)_2$ | CHCl | 3,4-Dichlorphenyl | |
| 541 | $C(CH_3)_2$ | CHCl | 2-Chlor-4-fluorphenyl | |
| 542 | $C(CH_3)_2$ | CHCl | 2-Trifluormethylphenyl | |
| 543 | $C(CH_3)_2$ | CHCl | 4-Trifluormethylphenyl | |
| 544 | $C(CH_3)_2$ | CHCl | 4-Methoxyphenyl | |
| 545 | $C(CH_3)_2$ | CHCl | 3,4-Dimethoxyphenyl | |
| 546 | $C(CH_3)_2$ | CHCl | 4-Tetrafluorethoxyphenyl | |
| 547 | $C(CH_3)_2$ | CHCl | 4-Trifluormethoxyphenyl | |
| 548 | $C(CH_3)_2$ | CHCl | 2-Chlor-4-(4'-Chlorphenoxy)phenyl | |

Tabelle (Fortsetzung)

| Nr. | A | B | Ar | phys. Daten IR, $cm^{-1}$ |
|---|---|---|---|---|
| 549 | $C(CH_3)_2$ | CHCl | 4-Phenoxyphenyl | |
| 550 | $C(CH_3)_2$ | CHCl | 4-(4'-Chlorphenoxy)phenyl | |
| 551 | $C(CH_3)_2$ | CHCl | 4-Benzyloxyphenyl | |
| 552 | $C(CH_3)(CH_2CH_3)$ | CHCl | Phenyl | |
| 553 | $C(CH_3)(CH_2CH_3)$ | CHCl | 2-Methylphenyl | |
| 554 | $C(CH_3)(CH_2CH_3)$ | CHCl | 4-Methylphenyl | |
| 555 | $C(CH_3)(CH_2CH_3)$ | CHCl | 2,4-Dimethylphenyl | |
| 556 | $C(CH_3)(CH_2CH_3)$ | CHCl | 2,6-Dimethylphenyl | |
| 557 | $C(CH_3)(CH_2CH_3)$ | CHCl | 2,4,6-Trimethylphenyl | |
| 558 | $C(CH_3)(CH_2CH_3)$ | CHCl | 4-tert. Butylphenyl | |
| 559 | $C(CH_3)(CH_2CH_3)$ | CHCl | 1-Naphthyl | |
| 560 | $C(CH_3)(CH_2CH_3)$ | CHCl | 2-Naphthyl | |
| 561 | $C(CH_3)(CH_2CH_3)$ | CHCl | 4-Biphenyl | |
| 562 | $C(CH_3)(CH_2CH_3)$ | CHCl | 2-Fluorphenyl | |
| 563 | $C(CH_3)(CH_2CH_3)$ | CHCl | 4-Fluorphenyl | |
| 564 | $C(CH_3)(CH_2CH_3)$ | CHCl | 2,4-Difluorphenyl | |
| 565 | $C(CH_3)(CH_2CH_3)$ | CHCl | 2-Chlorphenyl | |
| 566 | $C(CH_3)(CH_2CH_3)$ | CHCl | 3-Chlorphenyl | |
| 567 | $C(CH_3)(CH_2CH_3)$ | CHCl | 4-Chlorphenyl | |
| 568 | $C(CH_3)(CH_2CH_3)$ | CHCl | 2,4-Dichlorphenyl | |
| 569 | $C(CH_3)(CH_2CH_3)$ | CHCl | 3,4-Dichlorphenyl | |
| 570 | $C(CH_3)(CH_2CH_3)$ | CHCl | 2-Chlor-4-fluorphenyl | |
| 571 | $C(CH_3)(CH_2CH_3)$ | CHCl | 2-Trifluormethylphenyl | |
| 572 | $C(CH_3)(CH_2CH_3)$ | CHCl | 4-Trifluormethylphenyl | |
| 573 | $C(CH_3)(CH_2CH_3)$ | CHCl | 4-Methoxyphenyl | |

Tabelle (Fortsetzung)

| Nr. | A | B | Ar | phys. Daten | IR, $cm^{-1}$ |
|---|---|---|---|---|---|
| 574 | $C(CH_3)(CH_2CH_3)$ | CHCl | 3,4-Dimethoxyphenyl | | |
| 575 | $C(CH_3)(CH_2CH_3)$ | CHCl | 4-Tetrafluorethoxyphenyl | | |
| 576 | $C(CH_3)(CH_2CH_3)$ | CHCl | 4-Trifluormethoxyphenyl | | |
| 577 | $C(CH_3)(CH_2CH_3)$ | CHCl | 2-Chlor-4-(4'-Chlorphenoxy)phenyl | | |
| 578 | $C(CH_3)(CH_2CH_3)$ | CHCl | 4-Phenoxyphenyl | | |
| 579 | $C(CH_3)(CH_2CH_3)$ | CHCl | 4-(4'-Chlorphenoxy)phenyl | | |
| 580 | $C(CH_3)(CH_2CH_3)$ | CHCl | 4-Benzyloxyphenyl | | |
| 581 | $C(CH_2CH_3)_2$ | CHCl | Phenyl | Öl | 2967,2939,1478,1452,1423,1027 |
| 582 | $C(CH_2CH_3)_2$ | CHCl | 2-Methylphenyl | | 153-155°C |
| 583 | $C(CH_2CH_3)_2$ | CHCl | 4-Methylphenyl | | |
| 584 | $C(CH_2CH_3)_2$ | CHCl | 2,4-Dimethylphenyl | | |
| 585 | $C(CH_2CH_3)_2$ | CHCl | 2,6-Dimethylphenyl | | |
| 586 | $C(CH_2CH_3)_2$ | CHCl | 2,4,6-Trimethylphenyl | | 142-146°C |
| 587 | $C(CH_2CH_3)_2$ | CHCl | 4-tert. Butylphenyl | | |
| 588 | $C(CH_2CH_3)_2$ | CHCl | 1-Naphthyl | | |
| 589 | $C(CH_2CH_3)_2$ | CHCl | 2-Naphthyl | | |
| 590 | $C(CH_2CH_3)_2$ | CHCl | 4-Biphenyl | | |
| 591 | $C(CH_2CH_3)_2$ | CHCl | 2-Fluorphenyl | | |
| 592 | $C(CH_2CH_3)_2$ | CHCl | 4-Fluorphenyl | | |
| 593 | $C(CH_2CH_3)_2$ | CHCl | 2,4-Difluorphenyl | | |
| 594 | $C(CH_2CH_3)_2$ | CHCl | 2-Chlorphenyl | | |
| 595 | $C(CH_2CH_3)_2$ | CHCl | 3-Chlorphenyl | | |
| 596 | $C(CH_2CH_3)_2$ | CHCl | 4-Chlorphenyl | | |
| 597 | $C(CH_2CH_3)_2$ | CHCl | 2,4-Dichlorphenyl | | |
| 598 | $C(CH_2CH_3)_2$ | CHCl | 3,4-Dichlorphenyl | | |

Tabelle (Fortsetzung)

| Nr. | A | B | Ar | phys. Daten IR, $cm^{-1}$ |
|---|---|---|---|---|
| 599 | $C(CH_2CH_3)_2$ | CHCl | 2-Chlor-4-fluorphenyl | |
| 600 | $C(CH_2CH_3)_2$ | CHCl | 2-Trifluormethylphenyl | |
| 601 | $C(CH_2CH_3)_2$ | CHCl | 4-Trifluormethylphenyl | |
| 602 | $C(CH_2CH_3)_2$ | CHCl | 4-Methoxyphenyl | 87°C |
| 603 | $C(CH_2CH_3)_2$ | CHCl | 3,4-Dimethoxyphenyl | |
| 604 | $C(CH_2CH_3)_2$ | CHCl | 4-Tetrafluorethoxyphenyl | |
| 605 | $C(CH_2CH_3)_2$ | CHCl | 4-Trifluormethoxyphenyl | |
| 606 | $C(CH_2CH_3)_2$ | CHCl | 2-Chlor-4-(4'-Chlorphenoxy)phenyl | |
| 607 | $C(CH_2CH_3)_2$ | CHCl | 4-Phenoxyphenyl | |
| 608 | $C(CH_2CH_3)_2$ | CHCl | 4-(4'-Chlorphenoxy)phenyl | |
| 609 | $C(CH_2CH_3)_2$ | CHCl | 4-Benzyloxyphenyl | |
| 610 | Cyclopropylen | CHCl | Phenyl | |
| 611 | Cyclopropylen | CHCl | 2-Methylphenyl | |
| 612 | Cyclopropylen | CHCl | 4-Methylphenyl | |
| 613 | Cyclopropylen | CHCl | 2,4-Dimethylphenyl | |
| 614 | Cyclopropylen | CHCl | 2,6-Dimethylphenyl | |
| 615 | Cyclopropylen | CHCl | 2,4,6-Trimethylphenyl | |
| 616 | Cyclopropylen | CHCl | 4-tert. Butylphenyl | |
| 617 | Cyclopropylen | CHCl | 1-Naphthyl | |
| 618 | Cyclopropylen | CHCl | 2-Naphthyl | |
| 619 | Cyclopropylen | CHCl | 4-Biphenyl | |
| 620 | Cyclopropylen | CHCl | 2-Fluorphenyl | |
| 621 | Cyclopropylen | CHCl | 4-Fluorphenyl | |
| 622 | Cyclopropylen | CHCl | 2,4-Difluorphenyl | |
| 623 | Cyclopropylen | CHCl | 2-Chlorphenyl | |

Tabelle (Fortsetzung)

| Nr. | A | B | Ar | phys. Daten IR, $cm^{-1}$ |
|---|---|---|---|---|
| 624 | Cyclopropylen | CHCl | 3-Chlorphenyl | |
| 625 | Cyclopropylen | CHCl | 4-Chlorphenyl | |
| 626 | Cyclopropylen | CHCl | 2,4-Dichlorphenyl | |
| 627 | Cyclopropylen | CHCl | 3,4-Dichlorphenyl | |
| 628 | Cyclopropylen | CHCl | 2-Chlor-4-fluorphenyl | |
| 629 | Cyclopropylen | CHCl | 2-Trifluormethylphenyl | |
| 630 | Cyclopropylen | CHCl | 4-Trifluormethylphenyl | |
| 631 | Cyclopropylen | CHCl | 4-Methoxyphenyl | |
| 632 | Cyclopropylen | CHCl | 3,4-Dimethoxyphenyl | |
| 633 | Cyclopropylen | CHCl | 4-Tetrafluorethoxyphenyl | |
| 634 | Cyclopropylen | CHCl | 4-Trifluormethoxyphenyl | |
| 635 | Cyclopropylen | CHCl | 2-Chlor-4-(4'-Chlorphenoxy)phenyl | |
| 636 | Cyclopropylen | CHCl | 4-Phenoxyphenyl | |
| 637 | Cyclopropylen | CHCl | 4-(4'-Chlorphenoxy)phenyl | |
| 638 | Cyclopropylen | CHCl | 4-Benzyloxyphenyl | |
| 639 | Cyclopentylen | CHCl | Phenyl | |
| 640 | Cyclopentylen | CHCl | 2-Methylphenyl | 181-183°C |
| 641 | Cyclopentylen | CHCl | 4-Methylphenyl | |
| 642 | Cyclopentylen | CHCl | 2,4-Dimethylphenyl | |
| 643 | Cyclopentylen | CHCl | 2,6-Dimethylphenyl | |
| 644 | Cyclopentylen | CHCl | 2,4,6-Trimethylphenyl | 207-210°C |
| 645 | Cyclopentylen | CHCl | 4-tert. Butylphenyl | 155-158°C |
| 646 | Cyclopentylen | CHCl | 1-Naphthyl | |
| 647 | Cyclopentylen | CHCl | 2-Naphthyl | |
| 648 | Cyclopentylen | CHCl | 4-Biphenyl | |

Tabelle (Fortsetzung)

| Nr. | A | B | Ar | phys. Daten | IR, $cm^{-1}$ |
|---|---|---|---|---|---|
| 649 | Cyclopentylen | CHCl | 2-Fluorphenyl | | |
| 650 | Cyclopentylen | CHCl | 4-Fluorphenyl | öl | 2959,1606,1510,1478,1423,1228 |
| 651 | Cyclopentylen | CHCl | 2,4-Difluorphenyl | | |
| 652 | Cyclopentylen | CHCl | 2-Chlorphenyl | | |
| 653 | Cyclopentylen | CHCl | 3-Chlorphenyl | | |
| 654 | Cyclopentylen | CHCl | 4-Chlorphenyl | öl | 2959,2873,1492,1478,1423,1408 |
| 655 | Cyclopentylen | CHCl | 2,4-Dichlorphenyl | | |
| 656 | Cyclopentylen | CHCl | 3,4-Dichlorphenyl | | |
| 657 | Cyclopentylen | CHCl | 2-Chlor-4-fluorphenyl | | |
| 658 | Cyclopentylen | CHCl | 2-Trifluormethylphenyl | | |
| 659 | Cyclopentylen | CHCl | 4-Trifluormethylphenyl | | |
| 660 | Cyclopentylen | CHCl | 4-Methoxyphenyl | | |
| 661 | Cyclopentylen | CHCl | 3,4-Dimethoxyphenyl | | |
| 662 | Cyclopentylen | CHCl | 4-Tetrafluorethoxyphenyl | | |
| 663 | Cyclopentylen | CHCl | 4-Trifluormethoxyphenyl | | |
| 664 | Cyclopentylen | CHCl | 2-Chlor-4-(4'-Chlorphenoxy)phenyl | | |
| 665 | Cyclopentylen | CHCl | 4-Phenoxyphenyl | | |
| 666 | Cyclopentylen | CHCl | 4-(4'-Chlorphenoxy)phenyl | | |
| 667 | Cyclopentylen | CHCl | 4-Benzyloxyphenyl | | |
| 668 | Cyclohexylen | CHCl | Phenyl | | 230°C |
| 669 | Cyclohexylen | CHCl | 2-Methylphenyl | | |
| 670 | Cyclohexylen | CHCl | 4-Methylphenyl | | 213-216°C |
| 671 | Cyclohexylen | CHCl | 2,4-Dimethylphenyl | | |
| 672 | Cyclohexylen | CHCl | 2,6-Dimethylphenyl | | |
| 673 | Cyclohexylen | CHCl | 2,4,6-Trimethylphenyl | | |

Tabelle (Fortsetzung)

| Nr. | A | B | Ar | phys. Daten IR, $cm^{-1}$ |
|---|---|---|---|---|
| 674 | Cyclohexylen | CHCl | 4-tert. Butylphenyl | |
| 675 | Cyclohexylen | CHCl | 1-Naphthyl | |
| 676 | Cyclohexylen | CHCl | 2-Naphthyl | |
| 677 | Cyclohexylen | CHCl | 4-Biphenyl | |
| 678 | Cyclohexylen | CHCl | 2-Fluorphenyl | |
| 679 | Cyclohexylen | CHCl | 4-Fluorphenyl | 170-173°C |
| 680 | Cyclohexylen | CHCl | 2,4-Difluorphenyl | |
| 681 | Cyclohexylen | CHCl | 2-Chlorphenyl | |
| 682 | Cyclohexylen | CHCl | 3-Chlorphenyl | |
| 683 | Cyclohexylen | CHCl | 4-Chlorphenyl | 163°C |
| 684 | Cyclohexylen | CHCl | 2,4-Dichlorphenyl | |
| 685 | Cyclohexylen | CHCl | 3,4-Dichlorphenyl | |
| 686 | Cyclohexylen | CHCl | 2-Chlor-4-fluorphenyl | |
| 687 | Cyclohexylen | CHCl | 2-Trifluormethylphenyl | |
| 688 | Cyclohexylen | CHCl | 4-Trifluormethylphenyl | |
| 689 | Cyclohexylen | CHCl | 4-Methoxyphenyl | |
| 690 | Cyclohexylen | CHCl | 3,4-Dimethoxyphenyl | |
| 691 | Cyclohexylen | CHCl | 4-Tetrafluorethoxyphenyl | |
| 692 | Cyclohexylen | CHCl | 4-Trifluormethoxyphenyl | |
| 693 | Cyclohexylen | CHCl | 2-Chlor-4-(4'-Chlorphenoxy)phenyl | |
| 694 | Cyclohexylen | CHCl | 4-Phenoxyphenyl | |
| 695 | Cyclohexylen | CHCl | 4-(4'-Chlorphenoxy)phenyl | |
| 696 | Cyclohexylen | CHCl | 4-Benzyloxyphenyl | |
| 697 | $C(CH_3)(C_3H_7)$ | CHCl | Phenyl | |
| 698 | $C(CH_3)(C_3H_7)$ | CHCl | 2-Methylphenyl | |

Tabelle (Fortsetzung)

| Nr. | A | B | Ar | phys. Daten IR, $cm^{-1}$ |
|---|---|---|---|---|
| 699 | $C(CH_3)(C_3H_7)$ | CHCl | 4-Methylphenyl | |
| 700 | $C(CH_3)(C_3H_7)$ | CHCl | 2,4-Dimethylphenyl | |
| 701 | $C(CH_3)(C_3H_7)$ | CHCl | 2,6-Dimethylphenyl | |
| 702 | $C(CH_3)(C_3H_7)$ | CHCl | 2,4,6-Trimethylphenyl | |
| 703 | $C(CH_3)(C_3H_7)$ | CHCl | 4-tert. Butylphenyl | |
| 704 | $C(CH_3)(C_3H_7)$ | CHCl | 1-Naphthyl | |
| 705 | $C(CH_3)(C_3H_7)$ | CHCl | 2-Naphthyl | |
| 706 | $C(CH_3)(C_3H_7)$ | CHCl | 4-Biphenyl | |
| 707 | $C(CH_3)(C_3H_7)$ | CHCl | 2-Fluorphenyl | |
| 708 | $C(CH_3)(C_3H_7)$ | CHCl | 4-Fluorphenyl | |
| 709 | $C(CH_3)(C_3H_7)$ | CHCl | 2,4-Difluorphenyl | |
| 710 | $C(CH_3)(C_3H_7)$ | CHCl | 2-Chlorphenyl | |
| 711 | $C(CH_3)(C_3H_7)$ | CHCl | 3-Chlorphenyl | |
| 712 | $C(CH_3)(C_3H_7)$ | CHCl | 4-Chlorphenyl | |
| 713 | $C(CH_3)(C_3H_7)$ | CHCl | 2,4-Dichlorphenyl | |
| 714 | $C(CH_3)(C_3H_7)$ | CHCl | 3,4-Dichlorphenyl | |
| 715 | $C(CH_3)(C_3H_7)$ | CHCl | 2-Chlor-4-fluorphenyl | |
| 716 | $C(CH_3)(C_3H_7)$ | CHCl | 2-Trifluormethylphenyl | |
| 717 | $C(CH_3)(C_3H_7)$ | CHCl | 4-Trifluormethylphenyl | |
| 718 | $C(CH_3)(C_3H_7)$ | CHCl | 4-Methoxyphenyl | |
| 719 | $C(CH_3)(C_3H_7)$ | CHCl | 3,4-Dimethoxyphenyl | |
| 720 | $C(CH_3)(C_3H_7)$ | CHCl | 4-Tetrafluorethoxyphenyl | |
| 721 | $C(CH_3)(C_3H_7)$ | CHCl | 4-Trifluormethoxyphenyl | |
| 722 | $C(CH_3)(C_3H_7)$ | CHCl | 2-Chlor-4-(4'-Chlorphenoxy)phenyl | |
| 723 | $C(CH_3)(C_3H_7)$ | CHCl | 4-Phenoxyphenyl | |

Tabelle (Fortsetzung)

| Nr. | A | B | Ar | phys. Daten IR, $cm^{-1}$ |
|---|---|---|---|---|
| 724 | $C(CH_3)(C_3H_7)$ | CHCl | 4-(4'-Chlorphenoxy)phenyl | |
| 725 | $C(CH_3)(C_3H_7)$ | CHCl | 4-Benzyloxyphenyl | |
| 726 | $C(C_2H_5)(n-C_4H_9)$ | CHCl | Phenyl | |
| 727 | $C(C_2H_5)(n-C_4H_9)$ | CHCl | 2-Methylphenyl | |
| 728 | $C(C_2H_5)(n-C_4H_9)$ | CHCl | 4-Methylphenyl | |
| 729 | $C(C_2H_5)(n-C_4H_9)$ | CHCl | 2,4-Dimethylphenyl | |
| 730 | $C(C_2H_5)(n-C_4H_9)$ | CHCl | 2,6-Dimethylphenyl | |
| 731 | $C(C_2H_5)(n-C_4H_9)$ | CHCl | 2,4,6-Trimethylphenyl | |
| 732 | $C(C_2H_5)(n-C_4H_9)$ | CHCl | 4-tert. Butylphenyl | |
| 733 | $C(C_2H_5)(n-C_4H_9)$ | CHCl | 1-Naphthyl | |
| 734 | $C(C_2H_5)(n-C_4H_9)$ | CHCl | 2-Naphthyl | |
| 735 | $C(C_2H_5)(n-C_4H_9)$ | CHCl | 4-Biphenyl | |
| 736 | $C(C_2H_5)(n-C_4H_9)$ | CHCl | 2-Fluorphenyl | |
| 737 | $C(C_2H_5)(n-C_4H_9)$ | CHCl | 4-Fluorphenyl | |
| 738 | $C(C_2H_5)(n-C_4H_9)$ | CHCl | 2,4-Difluorphenyl | |
| 739 | $C(C_2H_5)(n-C_4H_9)$ | CHCl | 2-Chlorphenyl | |
| 740 | $C(C_2H_5)(n-C_4H_9)$ | CHCl | 3-Chlorphenyl | |
| 741 | $C(C_2H_5)(n-C_4H_9)$ | CHCl | 4-Chlorphenyl | |
| 742 | $C(C_2H_5)(n-C_4H_9)$ | CHCl | 2,4-Dichlorphenyl | |
| 743 | $C(C_2H_5)(n-C_4H_9)$ | CHCl | 3,4-Dichlorphenyl | |
| 744 | $C(C_2H_5)(n-C_4H_9)$ | CHCl | 2-Chlor-4-fluorphenyl | |
| 745 | $C(C_2H_5)(n-C_4H_9)$ | CHCl | 2-Trifluormethylphenyl | |
| 746 | $C(C_2H_5)(n-C_4H_9)$ | CHCl | 4-Trifluormethylphenyl | |
| 747 | $C(C_2H_5)(n-C_4H_9)$ | CHCl | 4-Methoxyphenyl | |
| 748 | $C(C_2H_5)(n-C_4H_9)$ | CHCl | 3,4-Dimethoxyphenyl | |

Tabelle (Fortsetzung)

| Nr. | A | B | Ar | phys. Daten IR, $cm^{-1}$ |
|---|---|---|---|---|
| 749 | $C(C_2H_5)(n-C_4H_9)$ | CHCl | 4-Tetrafluorethoxyphenyl | |
| 750 | $C(C_2H_5)(n-C_4H_9)$ | CHCl | 4-Trifluormethoxyphenyl | |
| 751 | $C(C_2H_5)(n-C_4H_9)$ | CHCl | 2-Chlor-4-(4'-Chlorphenoxy)-phenyl | |
| 752 | $C(C_2H_5)(n-C_4H_9)$ | CHCl | 4-Phenoxyphenyl | |
| 753 | $C(C_2H_5)(n-C_4H_9)$ | CHCl | 4-(4'-Chlorphenoxy)phenyl | |
| 754 | $C(C_2H_5)(n-C_4H_9)$ | CHCl | 4-Benzyloxyphenyl | |
| 755 | $CH(tert.C_4H_9)$ | CHCl | Phenyl | |
| 756 | $CH(tert.C_4H_9)$ | CHCl | 2-Methylphenyl | |
| 757 | $CH(tert.C_4H_9)$ | CHCl | 4-Methylphenyl | |
| 758 | $CH(tert.C_4H_9)$ | CHCl | 2,4-Dimethylphenyl | |
| 759 | $CH(tert.C_4H_9)$ | CHCl | 2,6-Dimethylphenyl | |
| 760 | $CH(tert.C_4H_9)$ | CHCl | 2,4,6-Trimethylphenyl | |
| 761 | $CH(tert.C_4H_9)$ | CHCl | 4-tert. Butylphenyl | |
| 762 | $CH(tert.C_4H_9)$ | CHCl | 1-Naphthyl | |
| 763 | $CH(tert.C_4H_9)$ | CHCl | 2-Naphthyl | |
| 764 | $CH(tert.C_4H_9)$ | CHCl | 4-Biphenyl | |
| 765 | $CH(tert.C_4H_9)$ | CHCl | 2-Fluorphenyl | |
| 766 | $CH(tert.C_4H_9)$ | CHCl | 4-Fluorphenyl | |
| 767 | $CH(tert.C_4H_9)$ | CHCl | 2,4-Difluorphenyl | |
| 768 | $CH(tert.C_4H_9)$ | CHCl | 2-Chlorphenyl | |
| 769 | $CH(tert.C_4H_9)$ | CHCl | 3-Chlorphenyl | |
| 770 | $CH(tert.C_4H_9)$ | CHCl | 4-Chlorphenyl | |
| 771 | $CH(tert.C_4H_9)$ | CHCl | 2,4-Dichlorphenyl | |
| 772 | $CH(tert.C_4H_9)$ | CHCl | 3,4-Dichlorphenyl | |
| 773 | $CH(tert.C_4H_9)$ | CHCl | 2-Chlor-4-fluorphenyl | |

Tabelle (Fortsetzung)

| Nr. | A | B | Ar | phys. Daten IR, $cm^{-1}$ |
|---|---|---|---|---|
| 774 | CH(tert.$C_4H_9$) | CHCl | 2-Trifluormethylphenyl | |
| 775 | CH(tert.$C_4H_9$) | CHCl | 4-Trifluormethylphenyl | |
| 776 | CH(tert.$C_4H_9$) | CHCl | 4-Methoxyphenyl | |
| 777 | CH(tert.$C_4H_9$) | CHCl | 3,4-Dimethoxyphenyl | |
| 778 | CH(tert.$C_4H_9$) | CHCl | 4-Tetrafluorethoxyphenyl | |
| 779 | CH(tert.$C_4H_9$) | CHCl | 4-Trifluormethoxyphenyl | |
| 780 | CH(tert.$C_4H_9$) | CHCl | 2-Chlor-4-(4'-Chlorphenoxy)phenyl | |
| 781 | CH(tert.$C_4H_9$) | CHCl | 4-Phenoxyphenyl | |
| 782 | CH(tert.$C_4H_9$) | CHCl | 4-(4'-Chlorphenoxy)phenyl | |
| 783 | CH(tert.$C_4H_9$) | CHCl | 4-Benzyloxyphenyl | |
| 784 | $C(CH_3)_2$ | CHOH | 3-Methylphenyl | 86-88°C |
| 785 | $C(CH_3)_2$ | CHOH | 2-Methoxyphenyl | 128-130°C |
| 786 | $C(CH_3)_2$ | CHOH | 3-Fluorphenyl | 3200, 2965, 1589, 1480, 1046 |
| 787 | $C(CH_3)_2$ | CHOH | 4-Isopropylphenyl | 97-98°C |
| 788 | $C(C_2H_5)_2$ | CHOH | 3-Methylphenyl | 3200, 2963, 1424, 1030, 716 |
| 789 | $C(C_2H_5)_2$ | CHOH | 3-Trifluormethylphenyl | 117-119°C |
| 790 | $C(C_2H_5)_2$ | CHOH | 2-Methoxyphenyl | 3200, 2962, 1488, 1238, 1030, 755 |
| 791 | $C(C_2H_5)_2$ | CHOH | 3-Fluorphenyl | 3200, 2966, 1589, 1480, 1258 |
| 792 | Cyclopentylen | CHOH | 3-Trifluormethylphenyl | 3200, 2956, 1329, 1163, 708 |
| 793 | Cyclopentylen | CHOH | 3-Methylphenyl | 3236, 2952, 1425, 1030, 717 |
| 794 | Cyclopentylen | CHOH | 2-Methoxyphenyl | 94-98°C |
| 795 | Cyclopentylen | CHOH | 4-tert.-Butylphenyl | 106-108°C |
| 796 | Cyclopentylen | CHOH | 3-Fluorphenyl | 3200, 2954, 1588, 1030, 717 |

Tabelle (Fortsetzung)

| Nr. | A | B | Ar | phys. Daten IR, $cm^{-1}$ |
|---|---|---|---|---|
| 797 | Cyclopentylen | CHOH | 4-Isopropylphenyl | 3227, 2957, 1424, 1030, 717 |
| 798 | Cyclohexylen | CHOH | 3-Methylphenyl | 137-139°C |
| 799 | Cyclohexylen | CHOH | 4-Isopropylphenyl | 98-100°C |
| 800 | Cyclohexylen | CHOH | 3-Trifluormethylphenyl | 3180, 2931, 1510, 1244, 1038, 713 |
| 801 | Cyclohexylen | CHOH | 2-Methoxyphenyl | 141-142°C |
| 802 | Cyclohexylen | CHOH | 3-Fluorphenyl | 146-148°C |
| 803 | $C(CH_3)_2$ | CHCl | 3-Methylphenyl | 144-146°C |
| 804 | $C(CH_3)_2$ | CHCl | 3-Fluorphenyl | 165-167°C |
| 805 | $C(CH_3)_2$ | CHCl | 4-Isopropylphenyl | 104-108°C |
| 806 | $C(C_2H_5)_2$ | CHCl | 3-Trifluormethylphenyl | 66-69°C |
| 807 | $C(C_2H_5)_2$ | CHCl | 3-Fluorphenyl | 205-207°C |
| 808 | $C(C_2H_5)_2$ | CHCl | 3-Methylphenyl | 107-110°C |
| 809 | $C(C_2H_5)_2$ | CHCl | 2-Methoxyphenyl | 83°C |
| 810 | Cyclopentylen | CHCl | 2-Methoxyphenyl | 142-143°C |
| 811 | $C(CH_3)_2$ | $C=NOCH_3$ | 4-Chlorphenyl | 47-49°C |
| 812 | $C(CH_3)_2$ | $C=NOCH_3$ | 4-Methylphenyl | 159-161°C |
| 813 | $C(CH_3)_2$ | $C=NOCH_3$ | 4-Fluorphenyl | 2970, 1507, 1423, 1224, 1067 |
| 814 | Cyclopentylen | $C=NOCH_3$ | 4-Fluorphenyl | 138-140°C |
| 815 | Cyclopentylen | CHF | 3-Trifluormethylphenyl | 1-H NMR ($DCl_3$): 2.95; 2.55 jeweils d, $^2J_{HH}$=15.5 Hz, 2H, $H_1$, 5.35d, $^2J_{FH}$=45.5Hz, 1H, $H_2$ |
| 816 | $C(CH_3)_2$ | CHF | 4-Fluorphenyl | 13 C-NMR ($DCl_3$): 39.34d, $^2J_{CF}$=21.4 Hz, C2, 41.6 d. $^3J_{CF}$=3.1, C1 98.62 d, $^1J_{CF}$=179.1 Hz C3 1H, $H_2$ |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze, die vor Pilzbefall zu schützenden Pflanzen, Saatgüter, Materialien oder der Erdboden mit einer fungizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anicnische Emulgatoren (z.B. Polyoxyethylen-Fettalkohol-Ether, Alkylsulfonate und Arylsulfonate) und Dispergiermittel, wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii. Bei der Saatgutbehandlung werden im allgemeinen Wirkstoffmengen von 0,001 bis 50 g, vorzugsweise 0,01 bis 10 g, je Kilogramm Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 3 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 12 werden in einer Mischung gelöst, die aus 40 Gew.-Teilen Cyclohexanon, 30 Gew.-Teilen Iso-butanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 16 werden in einer Mischung gelöst, die aus 25 Gew.-Teilen

Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 30 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 32 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 41 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 45 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 61 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde 1-Phenyl-3-(3-pyridinyl)-propan-1-on (A) - bekannt aus J.Org.Chem. Vol. 43 (1978) page 3396 - benutzt.

Anwendungsbeispiel 1

Wirksamkeit gegen Alternaria solani

Im Gewächshaus kultivierte Topfpflanzen der Sorte "Große Fleischtomate" wurden im Vierblattstadium mit wäßriger Suspension, die aus der Trockensubstanz - bestehend aus 80 % Wirkstoff und 20 % Emulgator - aufbereitet wurde, bis zur Tropfnässe besprüht. Nach dem Antrocknen des Spritzbelages wurden die Blätter mit einer wäßrigen Sporensuspension des Pilzes Alternaria solani inokuliert. Diese Pflanzen wurden dann in eine wasserdampfgesättigte Kammer mit Temperaturen zwischen 22 und 24°C gestellt. Nach 4 Tagen hatte sich die Krankheit auf den unbehandelten, jedoch inokulierten Pflanzen so stark entwickelt, daß die fungizide Wirksamkeit der Substanzen beurteilt werden konnte.

Das Ergebnis zeigt, daß die Wirkstoffe 1, 3, 12, 16, 30, 32, 41, 45, 61, 74, 117, 119, 128, 132, 133, 161, 175, 177, 186, 204, 206, 215, 219 bei der Anwendung als 0,05 %ige (Gew.%) Spritzbrühe eine bessere fungizide Wirkung zeigen (95 %) als der bekannte Vergleichswirkstoff A (10 %).

Anwendungsbeispiel 2

Wirksamkeit gegen Botrytis cinerea

Paprikasämlinge der Sorte "Neusiedler Ideal Elite" wurden, nachdem sich 4 bis 5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedecken.

Das Ergebnis zeigt, daß die Wirkstoffe 3, 16, 32, 45, 59, 61, 70, 132, 133, 177, 190, 206, 215, 219 bei der Anwendung als 0,05 %ige (Gew.%) Spritzbrühe eine bessere fungizide Wirkung Zeigen (95 %) als der

bekannte Vergleichswirkstoff A (30 %).

Anwendungsbeispiel 3

Wirksamkeit gegen Pyrenophora teres

Gerstenkeimlinge der Sorte "Igri" wurden im Zweikeimblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20 bis 22°C und 70 % relativer Luftfeuchtigkeit für weitere 5 Tage kultiviert. Dann wurde das Ausmaß des Blattbefalls ermittelt.

Das Ergebnis zeigt, daß die Wirkstoffe 1, 3, 12, 16, 32, 45, 61, 70, 74, 117, 119, 128, 132, 133, 157, 161, 177, 186, 204, 206, 215, 219, 650, 683 bei der Anwendung als 0,05 %ige (Gew.%) Spritzbrühe eine bessere fungizide Wirkung zeigen (95 %) als der bekannte Vergleichswirkstoff A (30 %).

**Patentansprüche**

**1.** β-Picolinderivate der Formel

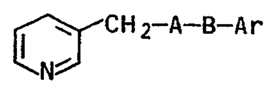

(I)

in der

A die Gruppe $CR^1R^2$ bedeutet, wobei

$R^1$, $R^2$ unabhängig voneinander für Wasserstoff, $C_1$-$C_6$-Alkyl, $C_2$-$C_6$-Alkenyl, $C_2$-$C_6$-Alkinyl stehen oder $R^1$ und $R^2$ zusammen eine Methylenkette mit 2 bis 6 Methylengruppen bedeuten,

B eine der Gruppen $CH_2$, $CHOR^3$, $CHR^4$, C=O, C=N-O-$R^5$ bedeutet, wobei

$R^3$ für Wasserstoff, $C_1$-$C_6$-Alkyl, $C_1$-$C_6$-Halogenalkyl, $C_3$-$C_8$-Cycloalkyl, $C_3$-$C_6$-Alkenyl, $C_2$-$C_6$-Acyl, Phenyl, Benzyl, Benzoyl steht, wobei der Phenylring ggf. durch ein bis drei Substituenten aus der Gruppe $C_1$-$C_4$-Alkyl, $C_1$-$C_4$-Alkoxy, $C_1$-$C_4$-Alkoxy, $C_1$-$C_4$-Halogenalkyl, $C_1$-$C_4$-Halogenalkoxy, Halogen, Cyan oder Nitro substituiert sein kann,

$R^4$ Wasserstoff, Fluor, Chlor, Brom oder Jod,

$R^5$ Wasserstoff, $C_1$-$C_6$-Alkyl, $C_1$-$C_6$-Halogenalkyl, $C_3$-$C_7$-Cycloalkyl, $C_3$-$C_6$-Alkenyl, Aralkyl mit 1 bis 4 Kohlenstoffatomen im Alkylrest bedeutet, wobei der Arylrest gegebenenfalls durch ein bis drei Substituenten aus der Gruppe $C_1$-$C_4$-Alkyl, $C_1$-$C_4$-Alkoxy, $C_1$-$C_4$-Halogenalkyl, Halogen, Cyan oder Nitro substituiert sein kann,

Ar einen ein- bis zweikernigen Arylrest bedeutet, der ggf. ein bis dreifach substituiert ist durch Halogen, $C_1$-$C_6$-Alkyl, $C_1$-$C_4$-Alkoxy, $C_1$-$C_4$-Halogenalkyl, $C_1$-$C_4$-Halogenalkoxy, Phenyl, Phenoxy, Halogenphenyl, Halogenphenoxy oder Benzyloxy,

und ihre N-Oxide und pflanzenverträglichen Säureadditionssalze, außer der Verbindung, in der A $CH_2$, B C=O und Ar Phenyl bedeuten.

**2.** Verfahren zur Herstellung von β-Picolinderivaten der Formel

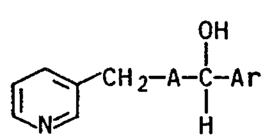

(Ia)

wobei A und Ar die im Anspruch 1 genannten Bedeutungen haben, dadurch gekennzeichnet, daß man

einen Aldehyd der Formel II

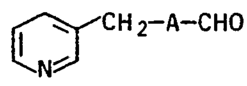

(II)

in der A die oben genannten Bedeutungen hat, umsetzt mit einer Organometallverbindung der Formel III

Ar-M (III)

in der M für Lithium oder einen der Reste MgCl, MgBr, MgJ steht und Ar die oben genannten Bedeutungen hat.

**3.** Verbindung der Formel II

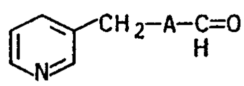

(II)

in der A die Gruppe $CR^1R^2$ bedeutet, wobei $R^1$, $R^2$ unabhängig voneinander für Wasserstoff, $C_1$-$C_6$-Alkyl, $C_2$-$C_6$-Alkenyl, $C_2$-$C_6$-Alkinyl stehen oder $R^1$ und $R^2$ zusammen eine Methylenkette mit 2 bis 6 Methylengruppen bedeutet, außer den Verbindungen, in denen $R^1$ Wasserstoff und $R^2$ Wasserstoff oder Methyl bedeuten.

**4.** Fungizid, enthaltend einen inerten Trägerstoff und eine fungizid wirksame Menge eines β-Picolinderivats der Formel

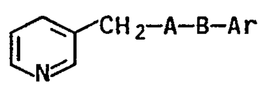

(I)

in der

A die Gruppe $CR^1R^2$ bedeutet, wobei

$R^1$, $R^2$ unabhängig voneinander für Wasserstoff, $C_1$-$C_6$-Alkyl, $C_2$-$C_6$-Alkenyl, $C_2$-$C_6$-Alkinyl stehen oder $R^1$ und $R^2$ zusammen eine Methylenkette mit 2 bis 6 Methylengruppen bedeuten,

B eine der Gruppen $CH_2$, $CHOR^3$, $CHR^4$, C = O, C = N-O-$R^5$ bedeutet, wobei

$R^3$ für Wasserstoff, $C_1$-$C_6$-Alkyl, $C_1$-$C_6$-Halogenalkyl, $C_3$-$C_8$-Cycloalkyl, $C_3$-$C_6$-Alkenyl, $C_2$-$C_6$-Acyl, Phenyl, Benzyl, Benzoyl steht, wobei der Phenylring ggf. durch ein bis drei Substituenten aus der Gruppe $C_1$-$C_4$-Alkyl, $C_1$-$C_4$-Alkoxy, $C_1$-$C_4$-Alkoxy, $C_1$-$C_4$-Halogenalkyl, $C_1$-$C_4$-Halogenalkoxy, Halogen, Cyan oder Nitro substituiert sein kann,

$R^4$ Wasserstoff, Fluor, Chlor, Brom oder Jod,

$R^5$ Wasserstoff, $C_1$-$C_6$-Alkyl, $C_1$-$C_6$-Halogenalkyl, $C_3$-$C_7$-Cycloalkyl, $C_3$-$C_6$-Alkenyl, Aralkyl mit 1 bis 4 Kohlenstoffatomen im Alkylrest bedeutet, wobei der Arylrest gegebenenfalls durch ein bis drei Substituenten aus der Gruppe $C_1$-$C_4$-Alkyl, $C_1$-$C_4$-Alkoxy, $C_1$-$C_4$-Halogenalkyl, Halogen, Cyan oder Nitro substituiert sein kann,

Ar einen ein- bis zweikernigen Arylrest bedeutet, der ggf. ein bis dreifach substituiert ist durch Halogen, $C_1$-$C_6$-Alkyl, $C_1$-$C_4$-Alkoxy, $C_1$-$C_4$-Halogenalkyl, $C_1$-$C_4$-Halogenalkoxy, Phenyl, Phenoxy, Halogenphenyl, Halogenphenoxy oder Benzyloxy,

oder ihres N-Oxids oder pflanzenverträglichen Säureadditionssalzes außer der Verbindung, in der A $CH_2$, B C = O und Ar Phenyl bedeuten.

5. Verfahren zur Bekämpfung von Pilzen, dadurch gekennzeichnet, daß man die Pilze oder die von Pilzbefall bedrohten Materialien, Pflanzen, Böden oder Saatgüter mit einer fungizid wirksamen Menge eines $\beta$-Picolinderivats der Formel I

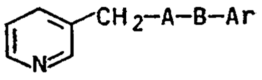

(I)

in der

A die Gruppe $CR^1R^2$ bedeutet, wobei

$R^1$, $R^2$ unabhängig voneinander für Wasserstoff, $C_1$-$C_6$-Alkyl, $C_2$-$C_6$-Alkenyl, $C_2$-$C_6$-Alkinyl stehen oder $R^1$ und $R^2$ zusammen eine Methylenkette mit 2 bis 6 Methylengruppen bedeuten,

B eine der Gruppen $CH_2$, $CHOR^3$, $CHR^4$, $C=O$, $C=N\text{-}O\text{-}R^5$ bedeutet, wobei

$R^3$ für Wasserstoff, $C_1$-$C_6$-Alkyl, $C_1$-$C_6$-Halogenalkyl, $C_3$-$C_8$-Cycloalkyl, $C_3$-$C_6$-Alkenyl, $C_2$-$C_6$-Acyl, Phenyl, Benzyl, Benzoyl steht, wobei der Phenylring ggf. durch ein bis drei Substituenten aus der Gruppe $C_1$-$C_4$-Alkyl, $C_1$-$C_4$-Alkoxy, $C_1$-$C_4$-Alkoxy, $C_1$-$C_4$-Halogenalkyl, $C_1$-$C_4$-Halogenalkoxy, Halogen, Cyan oder Nitro substituiert sein kann,

$R^4$ Wasserstoff, Fluor, Chlor, Brom oder Jod,

$R^5$ Wasserstoff, $C_1$-$C_6$-Alkyl, $C_1$-$C_6$-Halogenalkyl, $C_3$-$C_7$-Cycloalkyl, $C_3$-$C_6$-Alkenyl, Aralkyl mit 1 bis 4 Kohlenstoffatomen im Alkylrest bedeutet, wobei der Arylrest gegebenenfalls durch ein bis drei Substituenten aus der Gruppe $C_1$-$C_4$-Alkyl, $C_1$-$C_4$-Alkoxy, $C_1$-$C_4$-Halogenalkyl, Halogen, Cyan oder Nitro substituiert sein kann,

Ar einen ein- bis zweikernigen Arylrest bedeutet, der ggf. ein bis dreifach substituiert ist durch Halogen, $C_1$-$C_6$-Alkyl, $C_1$-$C_4$-Alkoxy, $C_1$-$C_4$-Halogenalkyl, $C_1$-$C_4$-Halogenalkoxy, Phenyl, Phenoxy, Halogenphenyl, Halogenphenoxy oder Benzyloxy,

oder ihres N-Oxids oder pflanzenverträgliches Säureadditionssalzes, behandelt.

6. Verbindung gemäß Anspruch 1, in der A $C(C_2H_5)_2$, B CHOH und Ar 4-Fluorphenyl bedeuten.

7. Verbindung gemäß Anspruch 1, in der A $C(C_2H_5)_2$, B $C=O$ und Ar 4-Fluorphenyl bedeuten.

8. Verbindung gemäß Anspruch 1, in der A $C(C_2H_5)_2$, B CHCl und Ar 4-Fluorphenyl bedeuten.